# EUROPEAN PATENT SPECIFICATION

(11) **EP 0 340 878 B1**
(45) Date of publication and mention of the grant of the patent: **16.05.2001**
(21) Application number: 89201173.5
(22) Date of filing: 08.05.1989
(51) Int. Cl.: C12N 15/53, C12N 9/02, C12P 33/00, C12N 1/19, C12N 1/21, C12N 5/10

(54) **Process for the biochemical oxidation of steroids and genetically engineered cells to be used therefor**
Verfahren zur biochemischen Oxidation von Steroiden sowie dafür verwendbare, genetisch manipulierte Zellen
Procédé de l'oxydation biochimique des stéroides et cellules génétiquement modifiées à cet usage

(30) Priority: 06.05.1988 EP 88200904; 23.09.1988 EP 88202080
(43) Date of publication of application: 08.11.1989
(73) Proprietor: Aventis Pharma S.A., 92160 Antony (FR)
(72) Inventor: Slijkhuis, Harmen, NL-2651 AE Berkel en Rodenrijs (NL); Selten, Gerardus Cornelis Maria, NL-2651 HZ Berkel en Rodenrijs (NL); Smaal, Erik Bastiaan, NL-2611 NS Delft (NL)

(56) References cited:
- US-A- 4 720 454
- PROC. NATL. ACAD. SCI. USA, vol. 84, no. 20, October 1987, pages 7193-7197, Washington, D.C., US; S.C. CHUA et al.: "Cloning of cDNA encoding steroid 11beta-hydroxylase (P450c11)
- PROC. NATL. ACAD. SCI. USA, vol. 81, September 1984, pages 5628-5632, Washington, D.C., US; M.E. JOHN et al.: "Identification and characterization of cDNA clones specific for cholesterol side-chain cleavage cytochrome P-450"
- BIOCHEMICAL AND BIOPHYSICAL RESEARCH COMMUNICATIONS, vol. 145, no. 3, 30th June 1987, pages 1239-1247, Academic Press, Inc.; Y. NONAKA et al.: "Molecular cloning and sequence analysis of full-length cDNA for mRNA of adrenodoxin oxidoreductase from bovine adrenal cortex"
- PROC. NATL. ACAD. SCI. USA, vol. 82, September 1985, pages 5705-5709, Washington D.C., US; T. OKAMURA et al.: "Molecular cloning and amino acid sequence of the precursor form of bovine adrenodoxin; Evidence for a previously unidentified COOH-terminal peptide
- SCIENCE, vol. 234, December 1986, pages 1258-1261, Washington, D.C., US; M.X. ZUBER et al.: "Expression of bovine 17alpha-hydroxylase cytochrome P-450 cDNA in nonsteroidogenic (COS 1) cells"
- PROC. NATL. ACAD. SCI. USA, vol. 81, April 1984, pages 1986-1990, Washington, D.C., US; P.C. WHITE et al.: "Cloning and expression of cDNA encoding a bovine adrenal cytochrome P-450 specific for steroid 21-hydroxylation
- OEDA ET AL.: "Expression of rat liver cytochrome P-450MC cDNA in Saccharomyces cerevisiae", DNA, , 1985, vol. 4, no. 3, pages 203 to 210

## Description

The invention relates to a biochemical oxidation process for the preparation of pharmaceutically useful steroids.

### BACKGROUND OF THE INVENTION

11β,17α,21-Trihydroxy-4-pregnene-3,20-dione (hydrocortisone) is an important pharmaceutical steroid, used for its pharmacological properties as a corticosteroid and as a starting compound for the preparation of numerous useful steroids, particularly other corticosteroids. Hydrocortisone is produced in the adrenal cortex of vertebrates and was originally obtained, in small amounts only, by a laborious extraction from adrenal cortex tissue. Only after structure elucidation new production routes were developed, characterised by a combination of chemical synthesis steps and microbiological conversions. Only because the starting compounds which are employed such as sterols, bile acids and sapogenins are abundant and cheap, the present processes afford a less expensive product, but these still are rather complicate. Several possibilities were envisaged to improve the present processes, and also biochemical approaches have been tried.

One attempt was to have a suited starting steroid converted in an in vitro biochemical system using the isolated adrenal cortex proteins which are known to be responsible for the enzymatical conversion in vivo of steroids to hydrocortisone. However, the difficult isolation of the proteins and the high price of the necessary cofactors, appeared to be prohibitive for an economically attractive large scale process. Another approach was to keep the catalysing proteins in their natural environment and to have the adrenal cortex cells produce in a cell culture the desired hydrocortisone. But due to the low productivity of the cells in practice it appeared to be impossible to make such a biochemical process economically attractive.

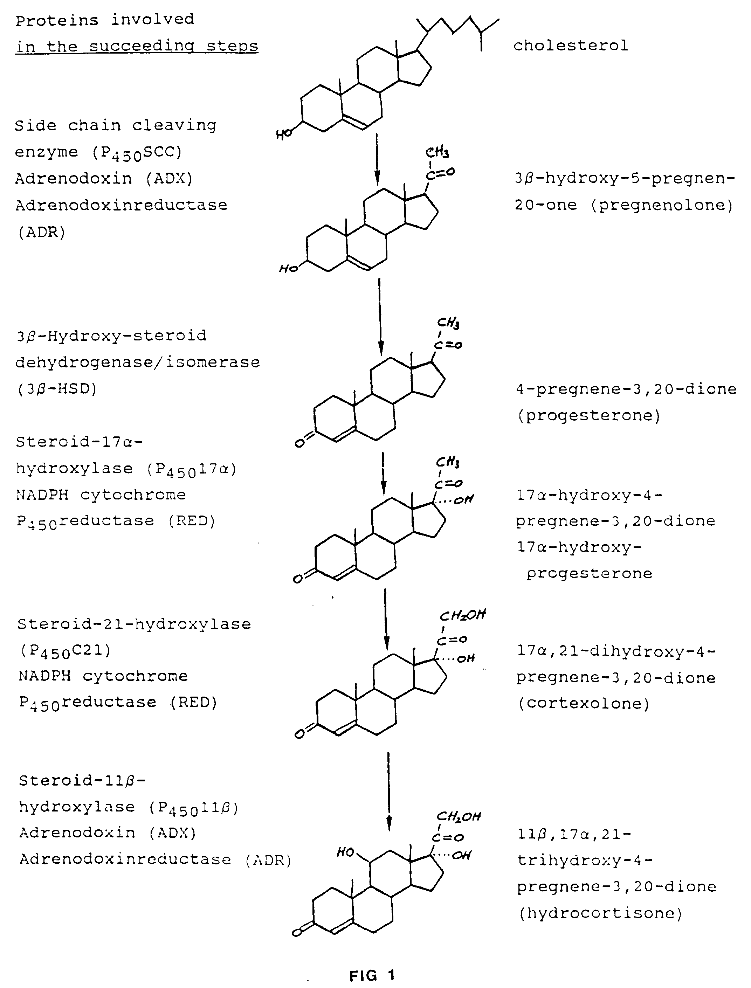

The in vivo process in the adrenal cortex of mammals and other vertebrates constitutes a biochemical pathway, which starts with cholesterol and via various intermediate compounds eventually affords hydrocortisone (see figure 1). Eight proteins are directly involved in this pathway, five of them being enzymes, among which four cytochrome P₄₅₀ enzymes, and the other three being electron transferring proteins.
The first step is the conversion of cholesterol to 3β-hydroxy-5-pregnen-20-one (pregnenolone). In this conversion, a mono-oxygenase reaction, three proteins are involved: side chain cleaving enzyme (P₄₅₀SCC, a heme-Fe-containing protein), adrenodoxin (ADX, a Fe₂S₂ containing protein) and adrenodoxinreductase (ADR, a FAD-containing protein). Besides cholesterol as a substrate the reaction further requires molecular oxygen and NADPH.
Subsequently pregnenolone is converted by dehydrogenation/ isomerisation to 4-pregnene-3,20-dione (progesterone). This reaction, catalysed by the protein 3β-hydroxysteroid dehydrogenase/isomerase (3β-HSD), requires pregnenolone and NAD⁺.
To obtain hydrocortisone progesterone subsequently is hydroxylated at three positions which conversions are catalysed by mono-oxygenases. In the conversion of progesterone into 17α-hydroxyprogesterone two proteins are involved:
steroid 17α-hydroxylase (P₄₅₀17α, a heme-Fe-containing protein) and NADPH cytochrome P₄₅₀reductase (RED, a FAD- and FMN-containing protein). The reaction consumes progesterone, molecular oxygen and NADPH.
For the conversion of 17α-hydroxyprogesterone into 17α,21-dihydroxy-4-pregnene-3,20-dione (cortexolone), also two proteins are needed: steroid-21-hydroxylase (P₄₅₀C21, a heme-Fe-containing protein) and the before-mentioned protein RED. The reaction consumes 17α-hydroxyprogesterone, molecular oxygen and NADPH.
In the conversion of cortexolone into hydrocortisone, three proteins are involved: steroid 11β-hydroxylase (P₄₅₀11β), a heme-Fe-containing protein, and the above-mentioned proteins ADX and ADR.

As described above cytochrome P₄₅₀ proteins are enzymes which are essential for the biochemical conversion of cholesterol to hydrocortisone. These enzymes belong to a larger group of cytochrome P₄₅₀ proteins (or shortly P₄₅₀ proteins). They have been encountered in prokaryotes (various bacteria) and eukaryotes (yeasts, moulds, plants and animals). In mammals high levels of P₄₅₀ proteins are found in the adrenal cortex, ovary, testes and liver. Many of these proteins have been purified and are well characterized now. Their specific activity has been determined. Recently a number of reviews on this subject have been published, such as K. Ruckpaul and H. Rein (eds), "Cytochrome P₄₅₀" and P.R. Ortiz de Montellano (ed.) "Cytochrome P₄₅₀, structure, mechanism and biochemistry". Cytochrome P₄₅₀ proteins are characterized by their specific absorbance maximum at 450 nm after reduction with carbon monoxide. In prokaryotic organisms the P₄₅₀ proteins are either membrane bound or cytoplasmatic. As far as the bacterial P₄₅₀ proteins have been studied in detail (e.g. P₄₅₀meg and P₄₅₀cam) it has been shown that a ferredoxin and a ferredoxinreductase are involved in the hydroxylating activity. For eukaryotic organisms, two types of P₄₅₀ proteins, I and II have been described. Their two differences reside in:
1. subcellular localisation, type I is localized in the microsomal fraction and type II is localized in the inner membrane of mitochondria;
2. the way the electrons are transferred to the P₄₅₀ protein. Type I is reduced by NADPH via a P₄₅₀reductase, whereas type II is reduced by NADPH via a ferredoxinreductase (e.g. adrenodoxinreductase) and a ferredoxin (e.g. adrenodoxin).

According to EP-A-0281245 cytochrome P₄₅₀ enzymes can be prepared from Streptomyces species and used for the hydroxylation of chemical compounds.
The enzymes are used in isolated form, which is a rather tedious and expensive procedure.
JP-A-62236485 (Derwent 87-331234) teaches that it is possible to introduce into Saccharomyces cerevisiae the genes of liver cytochrome P₄₅₀ enzymes and to express them affording enzymes which may be used for their oxidation activity.

However, in the above references there is no indication to the use of cytochrome P₄₅₀ enzymes for the preparation of steroid compounds.

### SUMMARY OF THE INVENTION

The invention provides a multiplicity of expression cassettes for production of proteins necessary in the construction of a multigenic system for the one-step conversion of inexpensive steroid starting materials to more rare and expensive end products, wherein such convertion is carried out in native systems through a multiplicity of enzyme-catalysed and cofactor-mediated conversions, such as the production of hydrocortisone from cholesterol. The expression cassettes of the invention are useful in the ultimate production of multigenic systems for conducting these multi-step conversions.

According, in one aspect, the invention is directed to the use of an expression cassette in multigenic systems for conducting multi-step conversions as depicted in figure 1, effective in a non mammalian recombinant host cell in expressing a heterologous coding DNA sequence, wherein said coding sequence encodes a protein which is functional, alone or in cooperation with one or more additional proteins, to catalyze an oxidation step in the biological pathway for to conversion of cholesterol to hydrocortisone. The expression cassettes of the invention, therefore, include those sequences capable of producting, in a recombinant host, the following proteins , side-chain cleaving enzyme (P₄₅₀SCC) ; adrenodoxin (ADX) ; adrenodoxin reductase (ADR) ; 3 β- hydroxysteroid dehydro-genase/isomerase (3β-HSD) ; steroid 17α-hydroxylase (P₄₅₀17α) ; NADPH cytochrome P₄₅₀ reductase (RED) ; steroid-21-hydroxylase (P₄₅₀C21) ; and steroid 11β-hydroxylase (P₄₅₀11β).

In other aspects, the invention is directed to recombinant host cells transformed with these vectors or with the expression cassettes of the invention, to methods to produce the above enzymes and to use these enzymes for oxidation, to processes to use said host cells for specific oxidations in a culture broth.

### BRIEF DESCRIPTION OF THE FIGURES

Abbrevations used in all figures : R₁, EcoRI ; H, HindIII; Sc, ScaI; P, PstI; K, KpnI; St, StuI; Sp, SphI; X, XbaI; N, NdeI; S, SmaI; Ss, SstI; Rᵥ, EcoRV; S_{I}, SaCI; B, BamHI; S_{II}, SacII; Sal, SALI; Xh, XhoI; Pv, PvuII; Bg, BglII; and M, MluI.

Figure 1 shows a schematic overview of the proteins involved in the succeeding steps in the conversion of cholesterol in hydrocortisone as occurring in the adrenal cortex of mammals.

Figure 2 shows the construction of plasmid pGBSCC-1. The P₄₅₀ SCC-sequences are indicated in a box( 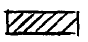 )

Figure 3 shows the insertion of a synthetically derived PstI/HindIII fragment containing the 5'-P₄₅₀SCC-sequences into the plasmid pTZ18R to abain the plasmid pTZ synlead.

Figure 4 shows the construction of a full-length P₄₅₀SCCcDNA of synthetically ( 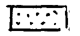 ) and by cDNA cloning- ( 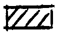 ) derived P₄₅₀ SCC-sequences into pTZ18R to obtain pGBSCC-2.

Figure 5 shows the complete nucleotide sequence of plasmid pBHA-1.

Figure 6 is a schematic representation of the construction of pGBSCC-3. P₄₅₀SCCcDNA sequences from plasmid pGBSCC-2 were introduced into the Bacillus/E.coli shuttle plasmid pBHA-1. Filled in boxes are as indicated in the legend of figure 4.

Figure 7 shows the introduction of a NdeI restriction site in combination with an ATG startcodon before the P₄₅₀SCC-maturation site in pGBSCC-3 to obtain pGBSCC-4.

Figure 8 shows a physical map of pGBSCC-5 which is obtained by removal of E.coli sequences from the plasmid pGBSCC-4.

Figure 9 shows a Western-blot probed with anti-bodies against P₄₅₀SCC, demonstrating the P₄₅₀SCC expression of plasmid pGBSCC-5 introduced in B.subtilis (lane c) and B.licheniformis (lane f). Control extracts from B.subtilis and B.licheniformis are shown in lane a and d, resp.. For comparison also purified adrenal cortex P₄₅₀SCC (30 ng) was added to these control extracts (lane b and e, resp.).

Figure 10 is a schematic representation of the construction of pGBSCC-17. The coding P₄₅₀SCC-DNA sequences from plasmid pGBSCC-4 were introduced into the E.coli expression vector pTZ18RN. The P₄₅₀SCC-sequences are indicated in a box ( 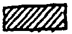 )

Figure 11 shows the P₄₅₀SCC expression of pGBSCC-17 in E.coli JM101.
(a) SDS/PAGE and Coomassie brilliant blue staining of the cellular protein fractions (20*µ*l) prepared from the E.coli control strain (lane 3) and E.coli transformants SCC-301 and 302 (lanes 1 and 2, resp.). 400 ng purified bovine P₄₅₀SCC (lane 4) is shown for comparison.
(b) Western-blot analysis probed with antibodies against P₄₅₀SCC of cellular protein fractions (5*µ*l) prepared from the control strain E.coli JM101 (lane 2) and from the E.coli transformants SCC-301 (lane 3) and SCC-302 (lane 4). 100 ng purified bovine P₄₅₀SCC (lane 1) is shown for comparison.

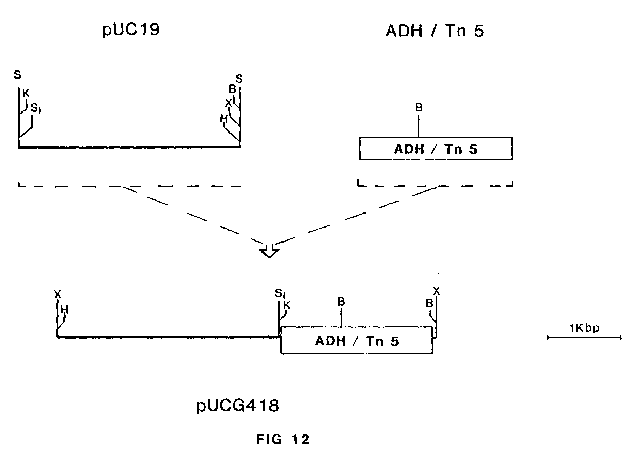

Figure 12 shows the construction of plasmid pUCG418.

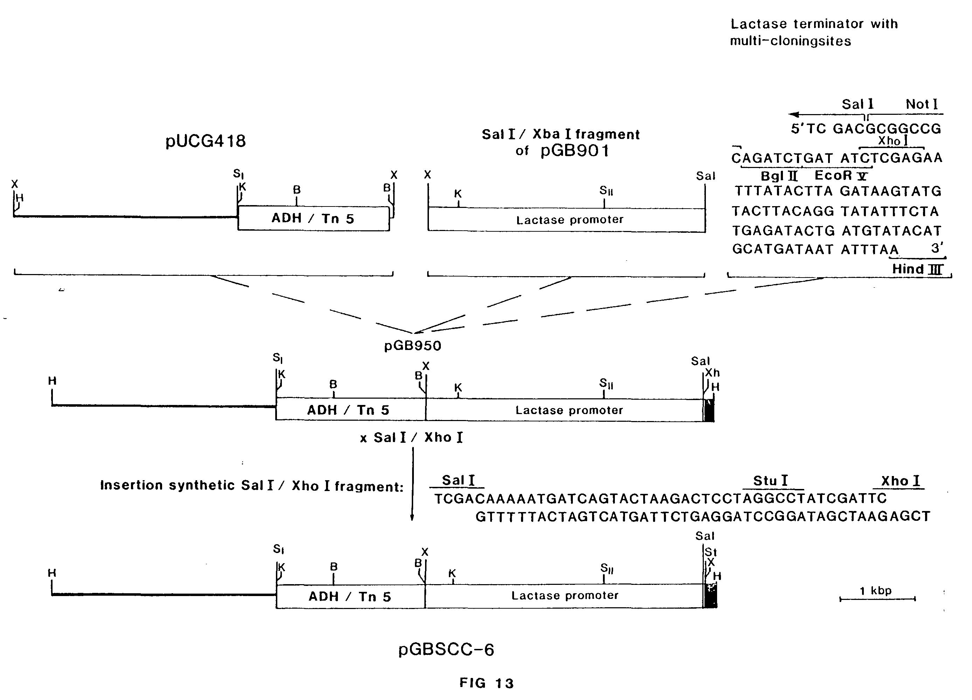

Figure 13 shows the construction of the yeast expression vector pGB950 by insertion of the promoter and terminator with multiple cloning sites ( 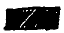 ) of lactase in pUCG418. To derive pGBSCC-6 a synthetic SalI/XhoI fragment containing an ATG startcodon and the codons for the first 8 amino acids of P₄₅₀SCC is inserted in pGB950.

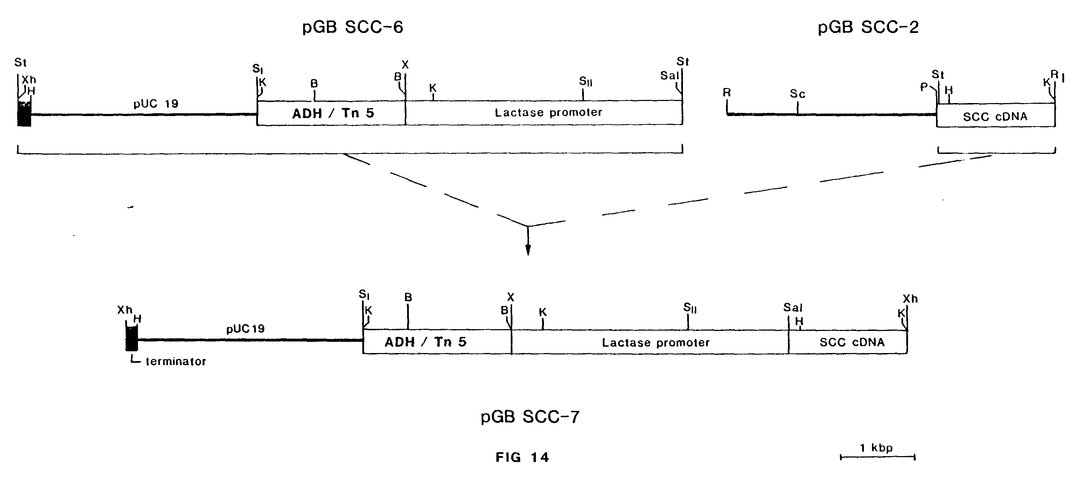

Figure 14 is a schematic presentation showing the construction of the yeast P₄₅₀SCC-expression cassette pGBSCC-7.

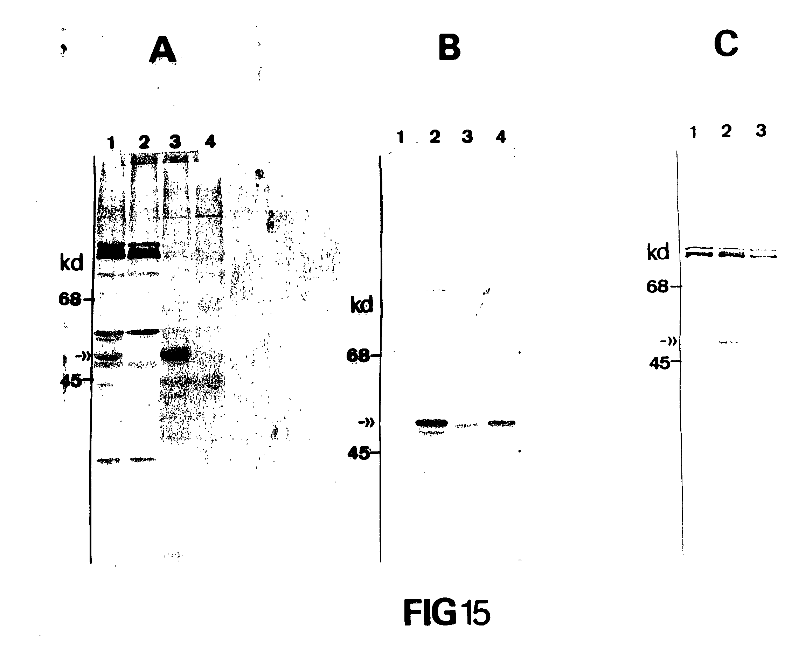

Figure 15 shows a Western-blot probed with antibodies specific for the protein P₄₅₀SCC.
Blot A contains extracts derived from Saccharomyces cerevisiae 273-10B transformed with pGBSCC-10 (lane 1); from S.cerevisiae 273-10B as a control (lane 2); from Kluyveromyces lactis CBS 2360 transformed with pGBSCC-7 (lane 3) and from K.lactis CBS 2360 as a control (lane 4). Blot B contains extracts derived from K.lactis CBS 2360 as a control (lane 1) and K.lactis CBS 2360 transformed with pGBSCC-15 (lane 2), with pGBSCC-12 (lane 3) or with pGBSCC-7 (lane 4). Blot C contains extracts derived from S.cerevisiae 273-10B as a control (lane 1), transformed with pGBSCC-16 (lane 2) or with pGBSCC-13 (lane 3).

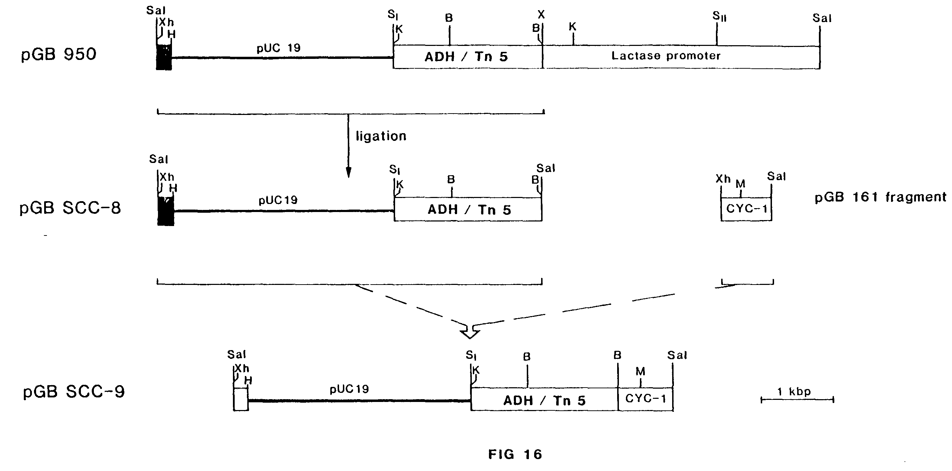

Figure 16 is a schematic presentation of the construction of the yeast expression vector pGBSCC-9 containing the isocytochrome CI (cyc-1) promoter from S.cerevisiae.

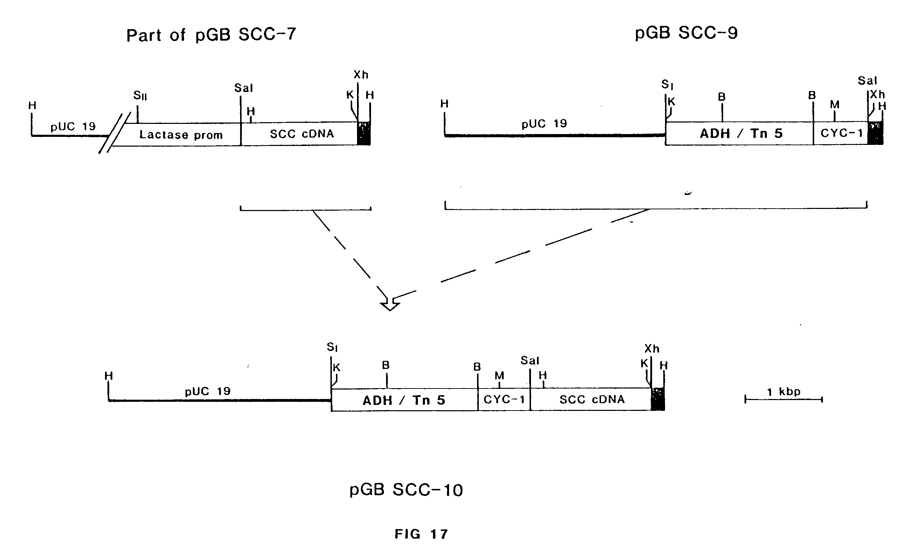

Figure 17 shows a construction diagram of the P₄₅₀SCCcDNA containing expression vector pGBSCC-10 for S.cerevisiae.

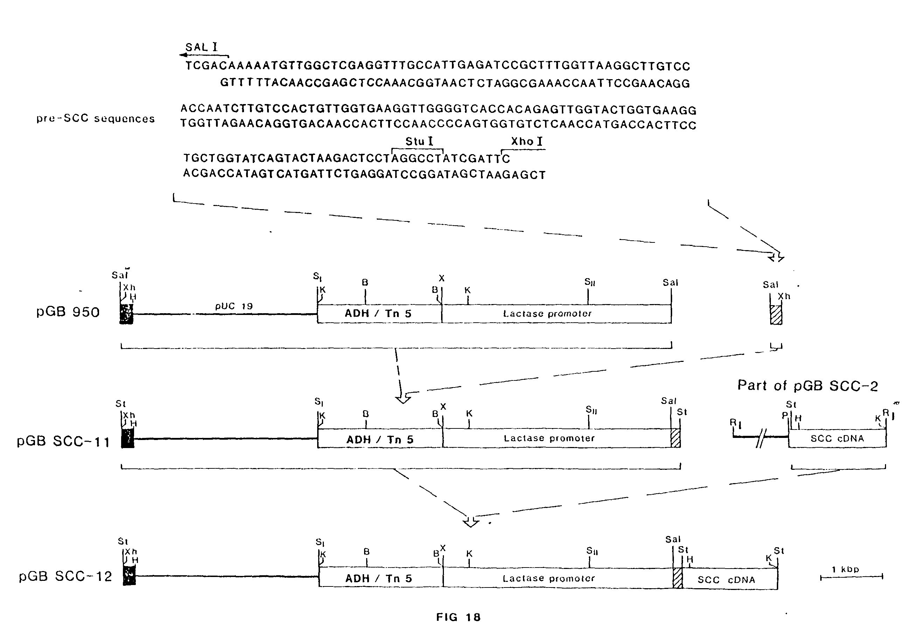

Figure 18 shows the construction of the P₄₅₀SCC expression vector pGBSCC-12 in which a synthetically derived DNA-fragment encoding the pre-P₄₅₀SCC sequence ( 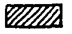 ) is inserted 5' for the coding sequence of mature P₄₅₀SCC.

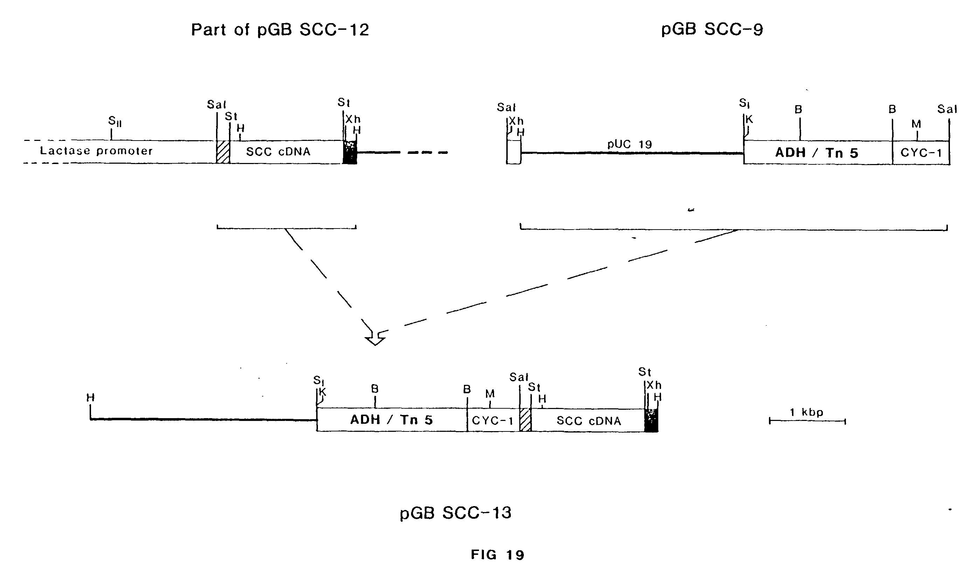

Figure 19 shows the construction of the pGBSCC-13. This P₄₅₀SCC expression cassette for S.cerevisiae contains the pre-P₄₅₀SCCcDNA sequence positioned 3' of the cyc-1 promoter of S.cerevisiae.

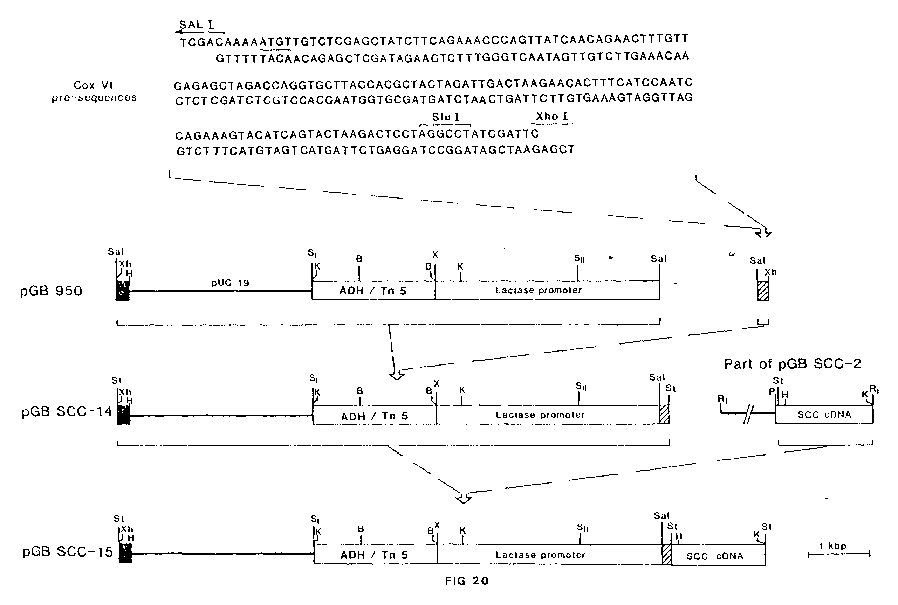

Figure 20 shows a schematic representation of the construction of the plasmids pGBSCC-14 and pGBSCC-15. The latter contains the P₄₅₀SCC coding sequence in frame with the cytochrome oxidase VI pre-sequence ( 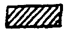 ).

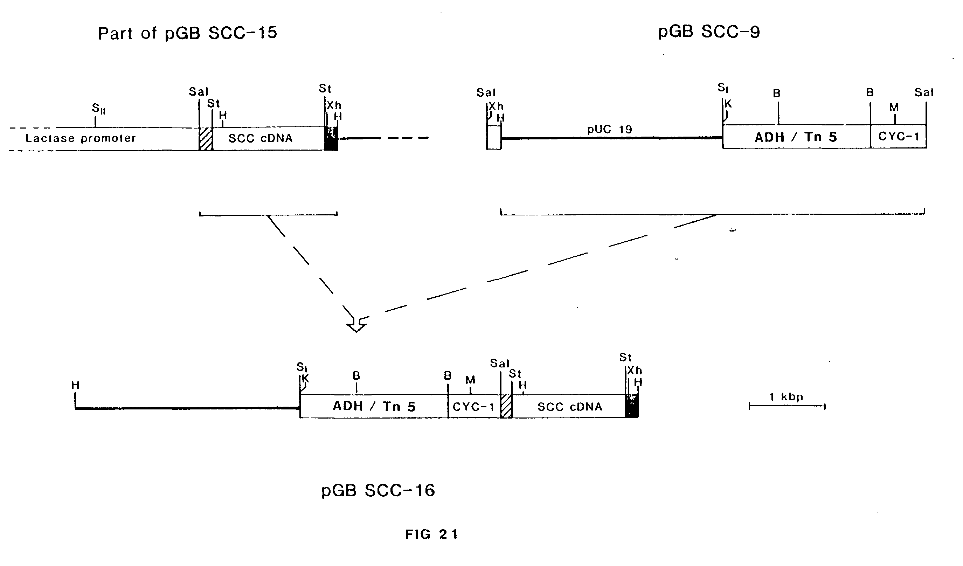

Figure 21 shows the construction of the plasmid pGBSCC-16. In this plasmid the cytochrome oxidase VI pre sequence 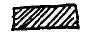 of S.cerevisiae fused to the coding P₄₅₀SCC sequence is positioned 3' of the cyc-1 promoter.

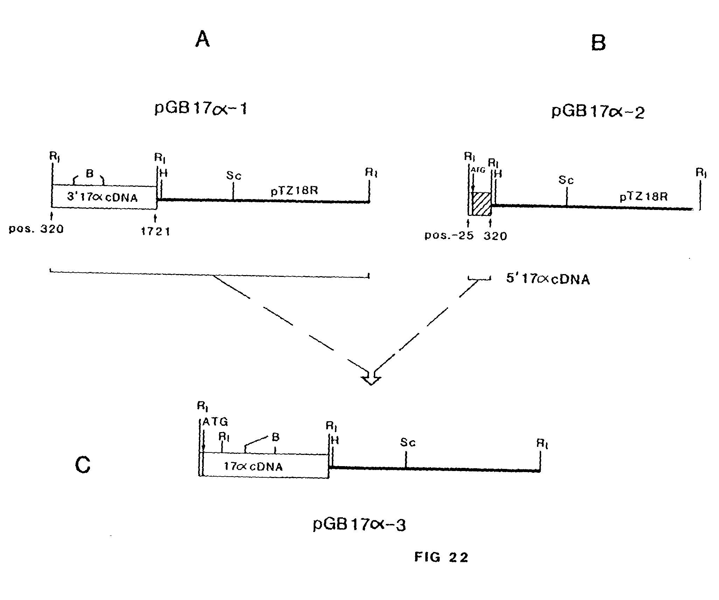

Figure 22 shows the physical maps of the plasmids pGB17α-1 (A) and pGB17α-2 (B) containing the 3' 1,4 kb fragment and the 5' 345 bp fragment 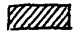 of P₄₅₀17αcDNA, resp.. In pGB17α-3 (C) containing the full length P₄₅₀17αcDNA sequence, the position of the ATG startcodon is indicated.

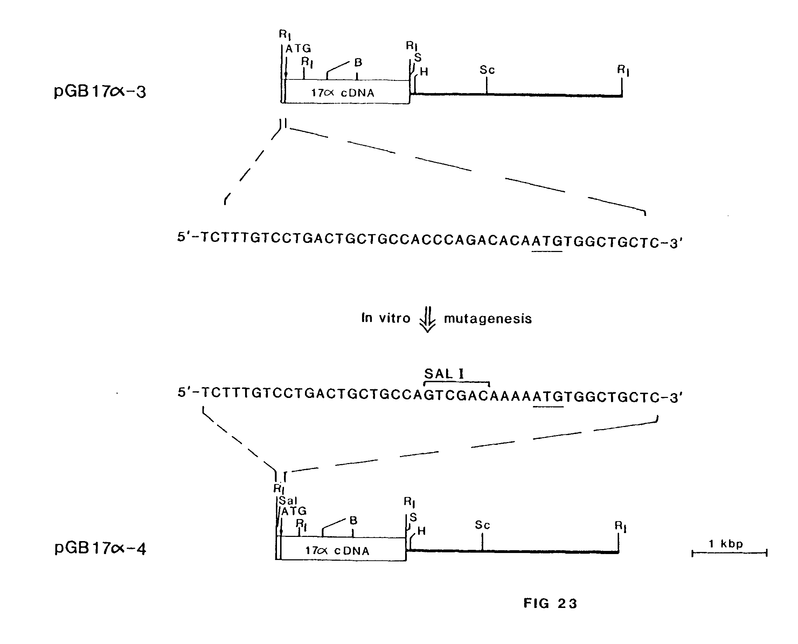

Figure 23 shows the mutation of pGB17α-3 by in vitro mutagenesis. The obtained plasmid pGB17α-4 contains a SalI restriction site followed by optimal yeast translation signals just upstream the ATG initiation codon.

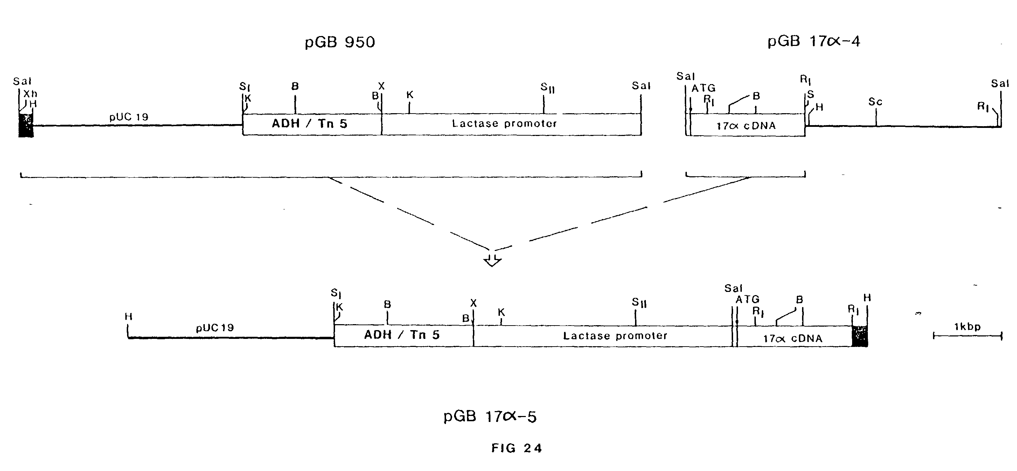

Figure 24 is a schematic view of the construction of the yeast P₄₅₀17α expression cassette pGB17α-5.

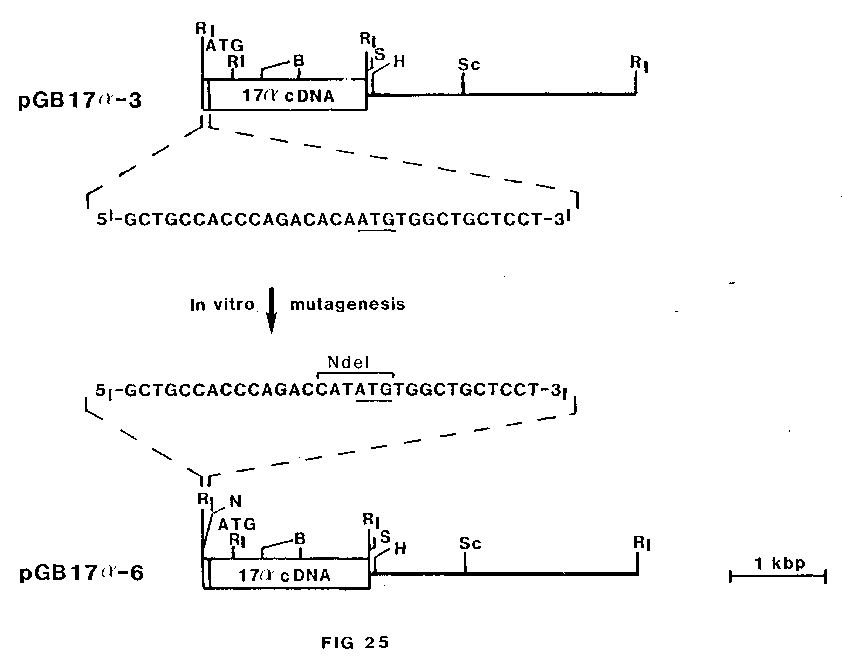

Figure 25 shows the mutation of pGB17α-3 by in vitro mutagenesis. The obtained plasmid pGB17α-6 contains an NdeI restriction site at the ATG-initiation codon.

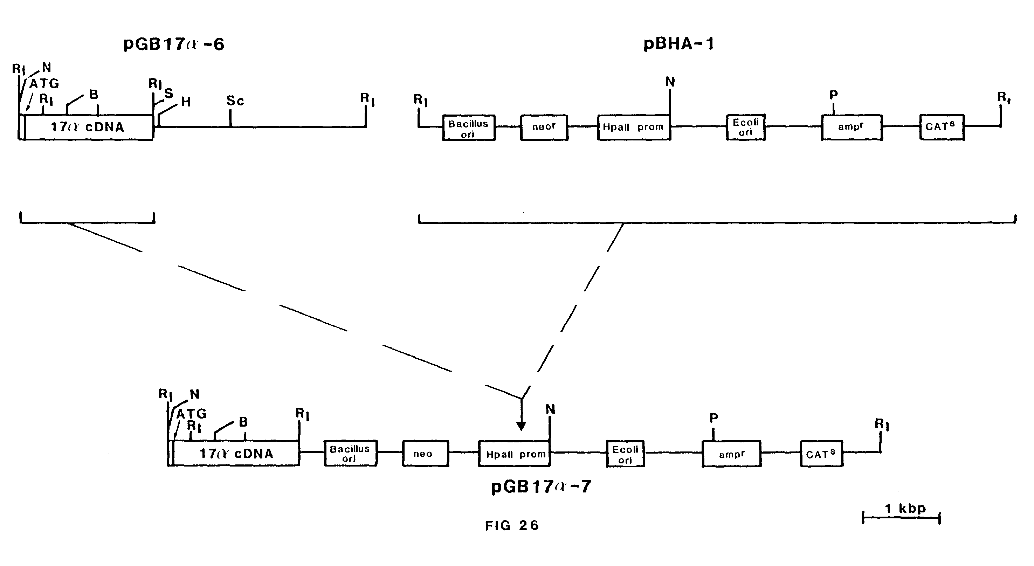

Figure 26 is a schematic representation of the construction of pGB17α-7. P₄₅₀17αcDNA sequences from plasmid pGB17α-6 were introduced into the Bacillus/E.coli shuttle plasmid pBHA-1.

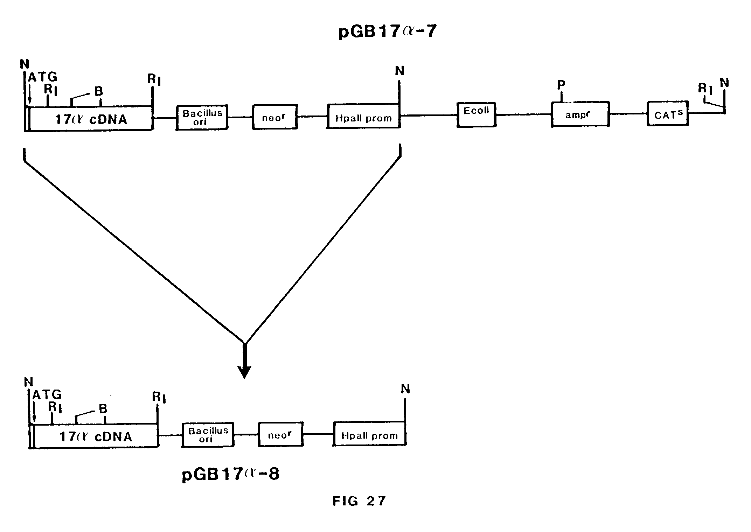

Figure 27 shows a physical map of pGB17α-8 which is obtained by removal of E.coli sequences from the plasmid pGB17α-7.

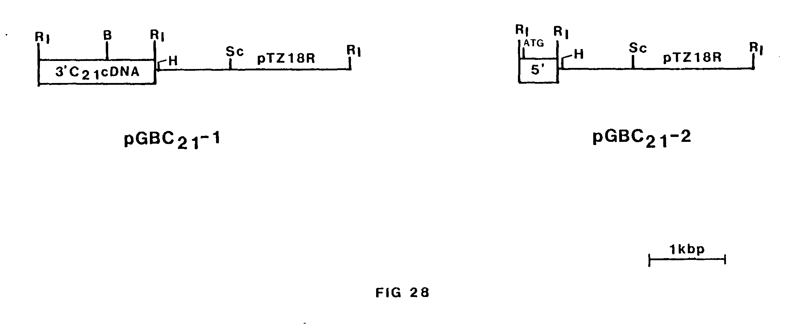

Figure 28 shows physical maps of pGBC21-1 and 2, containing an 1,53 Kb 3'-P₄₅₀C21cDNA and a 540 bp 5'-P₄₅₀C21cDNA EcoRI fragment, respectively, in the EcoRI-site of the cloning vector pTZ18R.

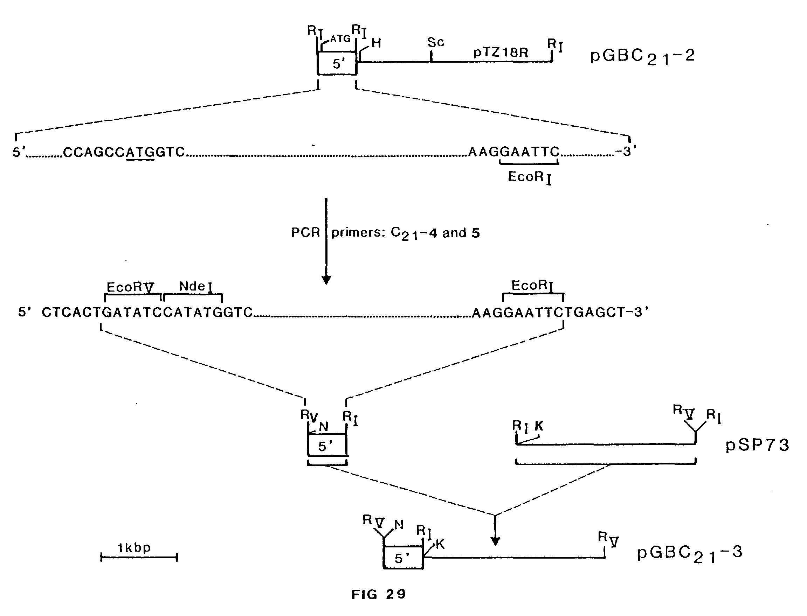

Figure 29 shows the in vitro mutagenesis by the polymerase chain reaction (PCR) of pGBC21-2 to introduce EcoRV and NdeI restriction sites upstream the P₄₅₀C21 ATG-initiation codon, followed by molecular cloning into the cloning vector pSP73 to derive pGBC21-3.

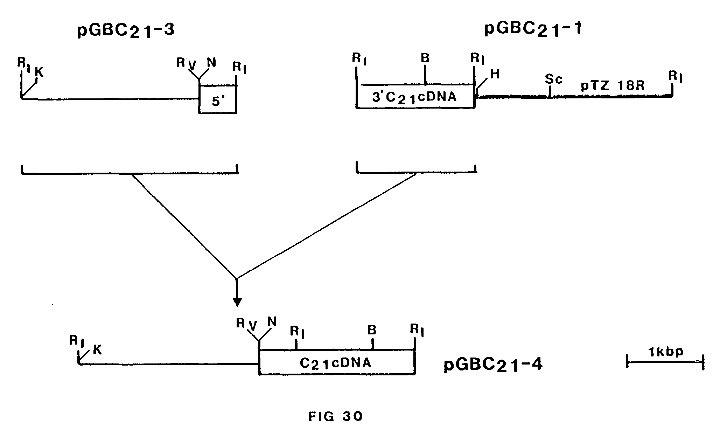

Figure 30 is a schematic view of the construction of pGBC21-4, containing the full-length P₄₅₀C21cDNA sequence.

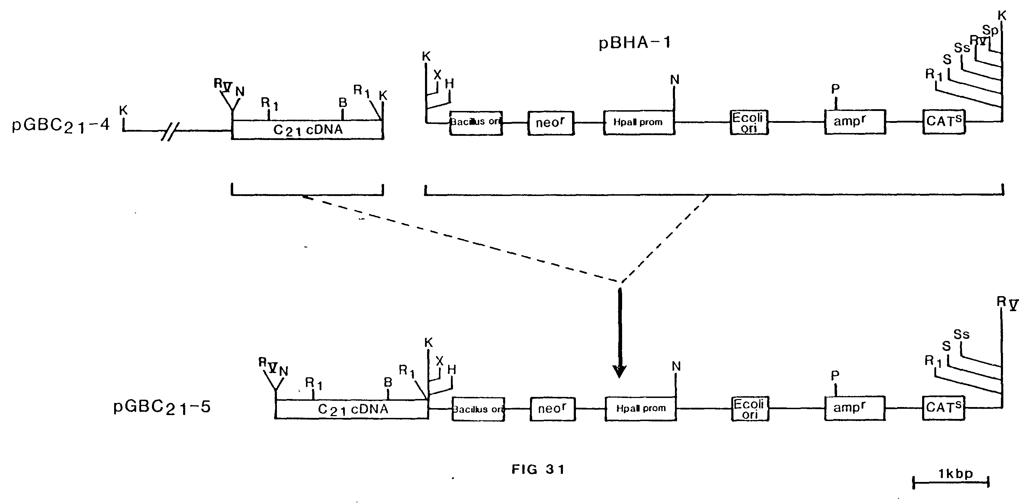

Figure 31 is a schematic representation of the construction of pGBC21-5. The P₄₅₀C21cDNA sequence from plasmid pGBC21-4 was introduced into the Bacillus/E.coli shuttle plasmid pBHA-1.

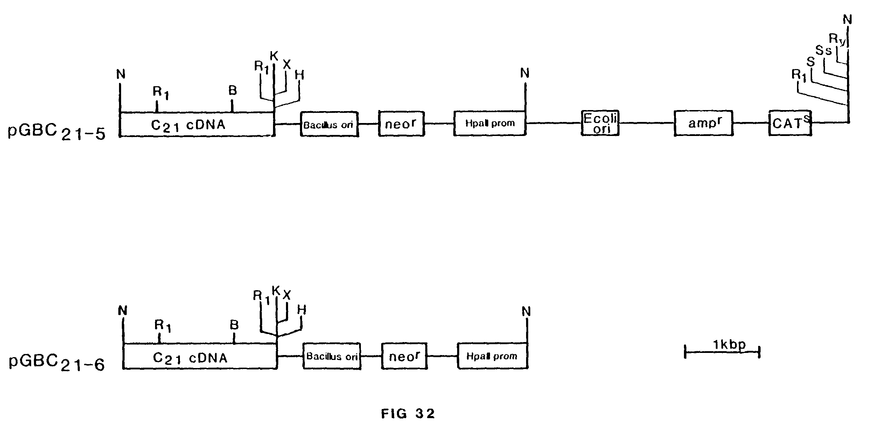

Figure 32 shows a physical map of pGBC21-6 which is obtained by removal of E.coli sequences from the plasmid pGBC21-5.

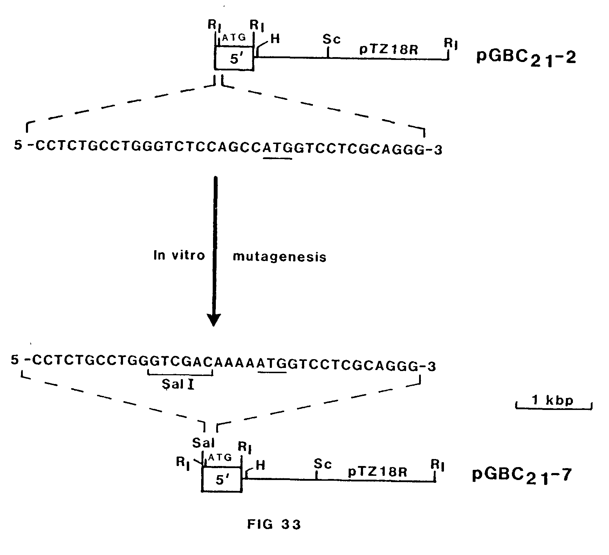

Figure 33 shows the mutation of pGBC21-2 by in vitro mutagenesis. The obtained plasmid pGBC21-7 contains a SalI restriction site followed by optimal yeast translation signals just upstream the ATG initiation codon.

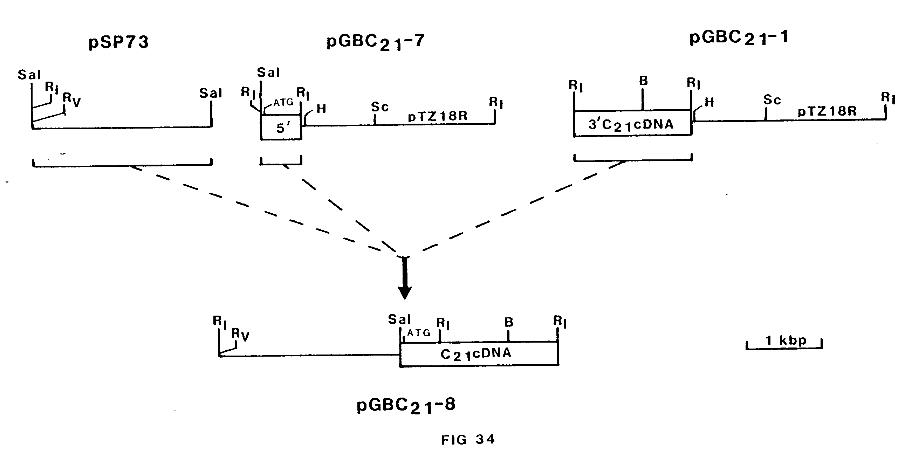

Figure 34 represents the construction of pGBC21-8, containing a full-length P₄₅₀C21cDNA with modified flanking restriction sites suitable for cloning into the yeast expression vector.

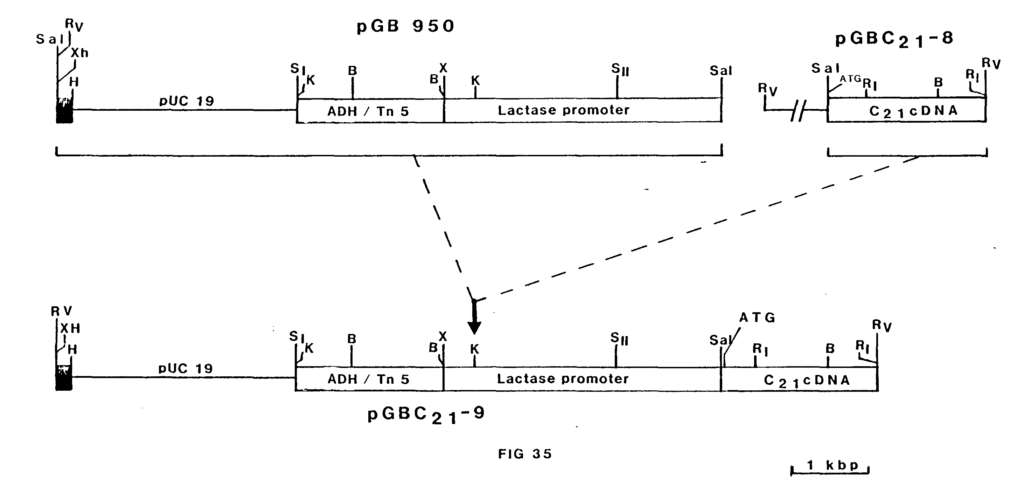

Figure 35 is a schematic presentation showing the construction of the yeast P₄₅₀C21-expression cassette pGBC21-9.

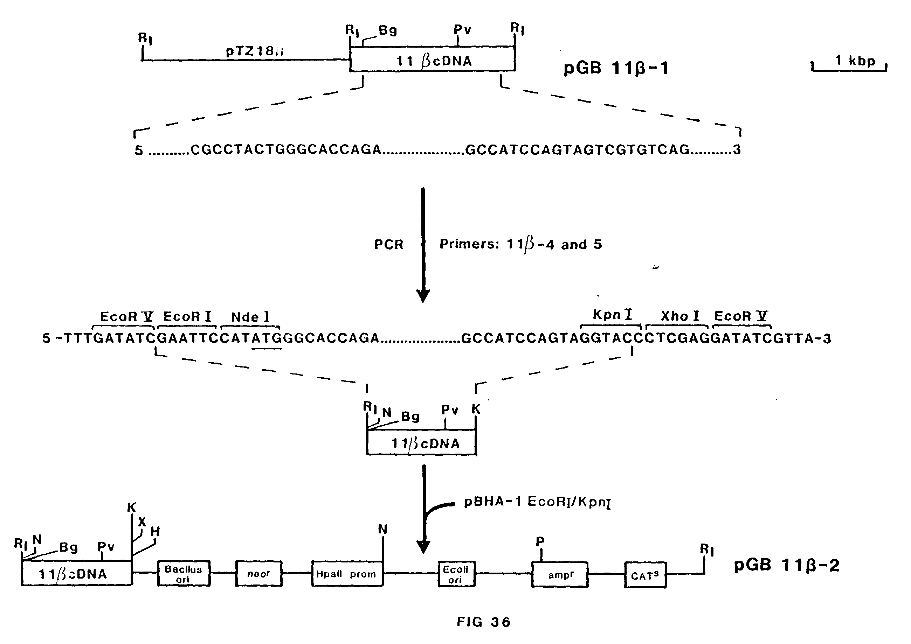

Figure 36 shows the in vitro mutagenesis by the polymerase chain reaction of pGB11β-1 to introduce appropriate flanking restriction sites and an ATG initiation codon to the full-length P₄₅₀11βcDNA sequence, followed by molecular cloning into the Bacillus/E.coli shuttle vector pBHA-1 to derive the plasmid pGB11β-2.

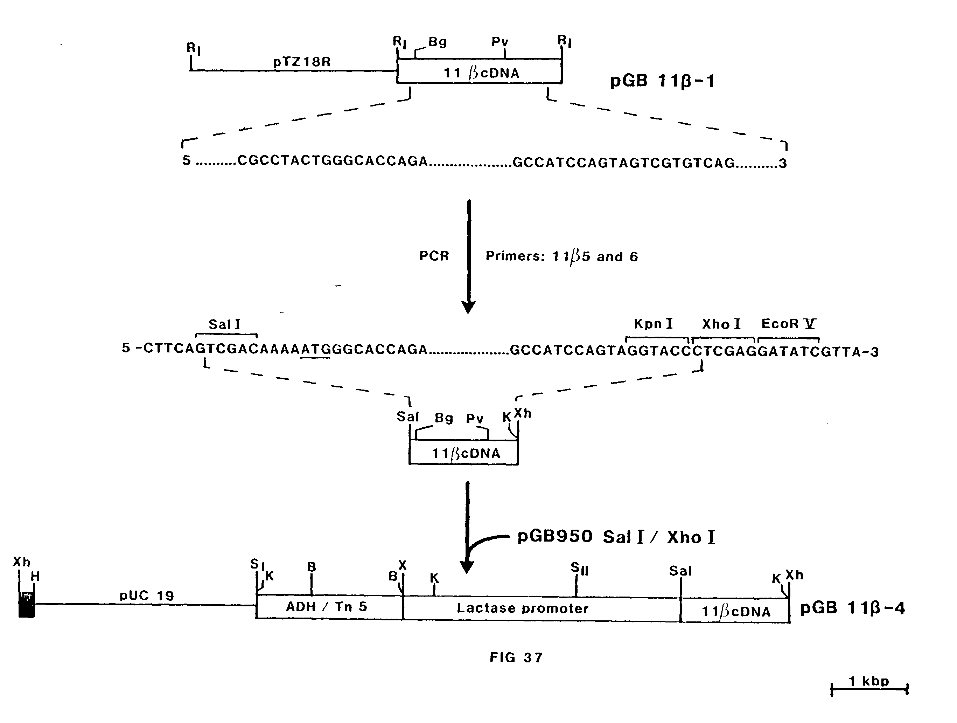

Figure 37 shows the in vitro mutagenesis by the polymerase chain reaction of pGB11β-1 to introduce appropriate flanking restriction sites and an ATG initation codon to the full-length P₄₅₀11βcDNA sequence, followed by molecular cloning into the yeast expression vector pGB950 to derive the plasmid pGB11β-4.

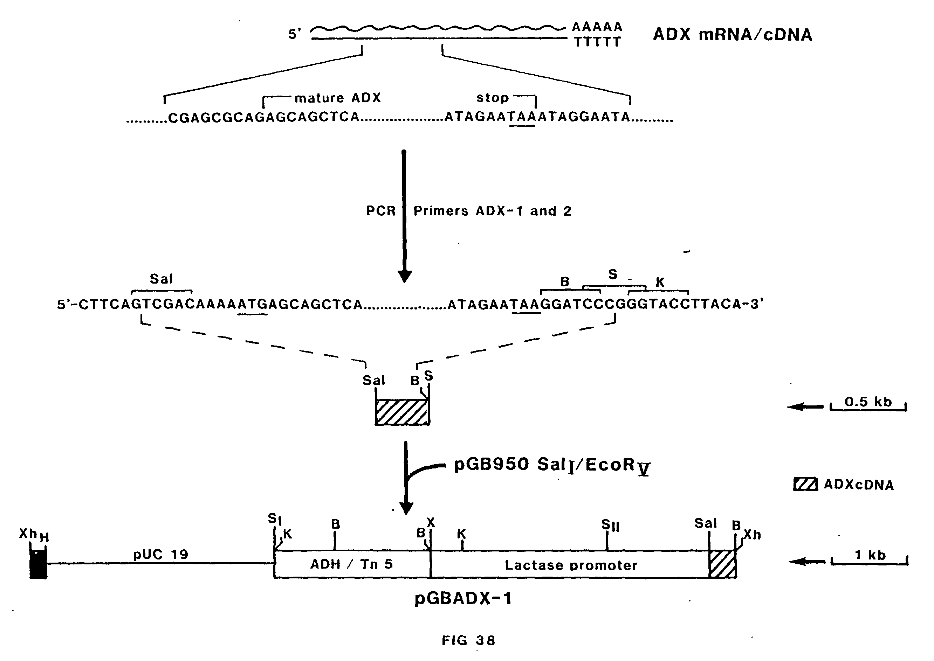

Figure 38 is a schematic view of the molecular cloning of the ADXcDNA sequence from a bovine adrenal cortex polyA⁺RNA/cDNA mixture by the polymerase chain reaction method. The cDNA sequence encoding the mature ADX protein was inserted into the appropriate sites of the yeast expression vector pGB950 to obtain the plasmid pGBADX-1.

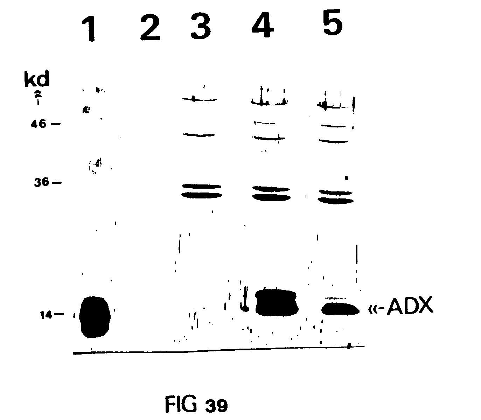

Figure 39 shows a Western-blot probed with anti-bodies against ADX, demonstrating the ADX expression of plasmid pGBADX-1 in K.lactis CBS 2360 transformants ADX-101 and 102 (lanes 4 and 5, resp.). Extract of control strain K.lactis CBS 2360 is shown in lane 3. For comparison also purified adrenal cortex ADX (100 ng) is supplied to the gel in lane 1.

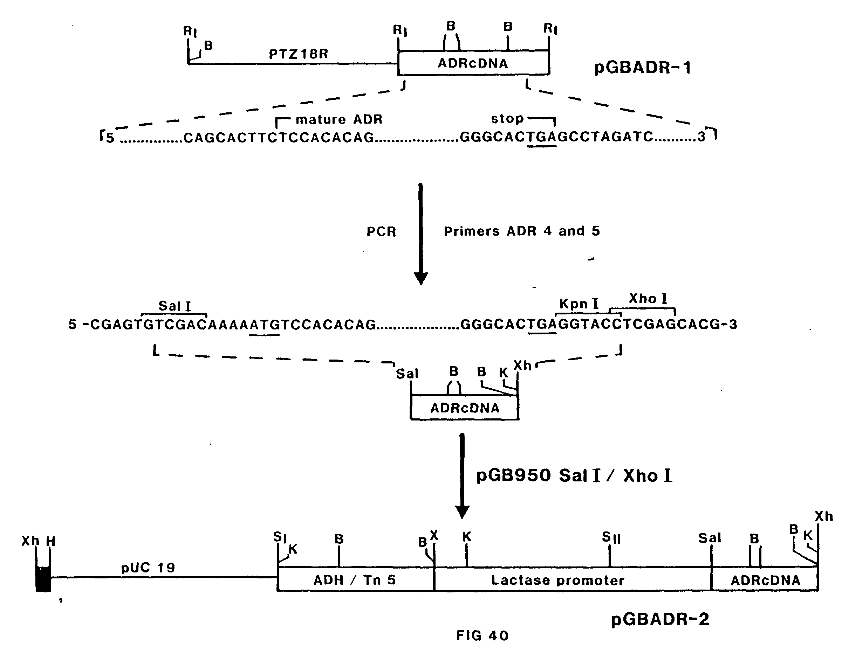

Figure 40 shows the in vitro mutagenesis by the polymerase chain reaction of pGBADR-1 to introduce appropriate flanking restriction sites and an ATG-initation codon to the full-length ADRcDNA sequence, followed by molecular cloning into the yeast expression vector pGB950 to derive pGBADR-2.

### DETAILS OF THE INVENTION

The invention comprises the preparation and culturing of cells which are suited to be employed in large scale biochemical production reactors and the use of these cells for the oxidation of compounds and particularly for the production of steroids, shown in figure 1. Interchange of steps in a multi-step reaction is included in the invention. Micro-organisms are preferred hosts but other cells may be used as well, such as cells of plants or non mammalian animals, optionally applied in a cell culture or in the tissue of living transgenic plants or non mammalian animals.

The cells according to the invention are obtained by the genetic transformation of suitable receptor cells, preferably cells of suited micro-organisms, with vectors containing DNA sequences encoding the proteins involved in the conversion of cholesterol to hydrocortisone, comprising side-chain cleaving enzyme (P₄₅₀SCC), adrenodoxin (ADX), adrenodoxin reductase (ADR), 3β-hydroxy-steroid dehydrogenase/isomerase (3β-HSD), steroid-17α-hydroxylase (P₄₅₀17α), NADPH cytochrome P₄₅₀ reductase (RED), steroid-21-hydroxylase (P₄₅₀C21) and steroid-11β-hydroxylase (P₄₅₀11β). Some host cells may already produce of their own one or more of the necessary proteins at a sufficient level and therefore have to be transformed with the supplementary DNA sequences only. Such possibly own proteins are ferredoxin, ferredoxin reductase, P₄₅₀-reductase, and 3β-hydroxy-steroid dehydrogenase/isomerase.

For retrieval of the sequences which encode proteins which are involved in the conversion of cholesterol to hydrocortisone, suitable DNA sources have been selected. An appropriate source for the retrieval of DNA encoding all proteins involved in the conversion of cholesterol to hydrocortisone is the adrenal cortex tissue of vertebrates e.g. bovine adrenal cortex tissue. Also from various micro-organisms, the relevant DNA can be retrieved, e.g. from Pseudomonas testosteroni, Streptomyces griseocarneus or Brevibacterium sterolicum for DNA encoding the 3β-hydroxysteroid dehydrogenase/isomerase and from Curvularia lunata or Cunninghamella blakesleeana for DNA encoding proteins involved in the 11β-hydroxylation of cortexolone. The DNA-sequences coding for the proteins bovine P₄₅₀SCC, bovine P₄₅₀11β or a microbial equivalent protein, bovine adrenodoxin, bovine adrenodoxin reductase, 3β-hydroxy-steroid dehydrogenase/isomerase of bovine or microbial origin, bovine P₄₅₀17α, bovine P₄₅₀C21 and NADPH cytochrome P₄₅₀ reductase of bovine or microbial origin, were isolated according to the following steps:

### 1. Eukaryotic sequences (cDNA's)

a. Total RNA was prepared from appropriate tissue
b. PolyA⁺ containing RNA was transcribed into double stranded cDNA and ligated into bacteriophage vectors
c. The obtained cDNA library was screened with ³²P-labeled oligomers specific for the desired cDNA or by screening an isopropyl-β-D-thiogalactopyranoside (IPTG)-induced lambda-gt11 cDNA library using a specific (¹²⁵I-labeled) antibody
d. cDNA inserts of positive plaque forming units (pfu's) were inserted into appropriate vectors to verify:
   - the entire length of the cDNA by nucleotide sequencing

### 2. Prokaryotic genes

a. Genomic DNA was prepared from an appropriate microorganism
b. To obtain a DNA library DNA fragments were cloned into appropriate vectors and transformed to an appropriate E.coli host
c. The DNA library was screened with ³²P-labeled oligomers specific for the gene of interest or by screening an IPTG-induced lambda-gtll DNA library using a specific (¹²⁵I-labeled) antibody
d. Plasmids of positive colonies were isolated and inserted DNA fragments subcloned into appropriate vectors to verify:
   - the entire length of the gene
Note: according to an a improved method the particular cDNA (eukaryotic sequences) or gene (prokaryotic sequences) was amplified using two specific oligomers by the method known as the polymerase chain reaction (PCR) (Saiki et al, Science, 239, 487-491, 1998). Subsequently the amplified cDNA or DNA was inserted into the appropriate vectors.

According to one aspect of the invention suitable expression cassettes are provided in which the heterologous DNA isolated according to the previous procedure and encoding an enzyme which catalyzes an oxydation step, of the pathway of figure 1 and at least one additional protein of said pathway, is placed between suitable control sequences for transcription and translation, which enables the DNA to be expressed in the cellular environment of a suitable host, affording the desired proteins. Optionally, the initiation control sequences are followed by a secretion signal sequence.

Suitable control sequences have to be introduced together with the structural DNA by said expression cassettes. Expression is made possible by transformation of a suitable host cell with a vector containing control sequences which are compatible with the relevant host and are in operable linkage to the coding sequences of which expression is desired.
Alternatively, suitable control sequences present in the host genome are employed. Expression is made possible by transformation of a suitable host cell with a vector containing coding sequences of the desired proteins flanked by host sequences enabling homologous recombination with the host genome in such a manner that host control sequences properly control the expression of the introduced DNA.

As is generally understood, the term control sequences comprises all DNA segments which are necessary for the proper regulation of the expression of the coding sequence to which they are operably linked, such as operators, enhancers and, particularly, promoters and sequences which control the translation.

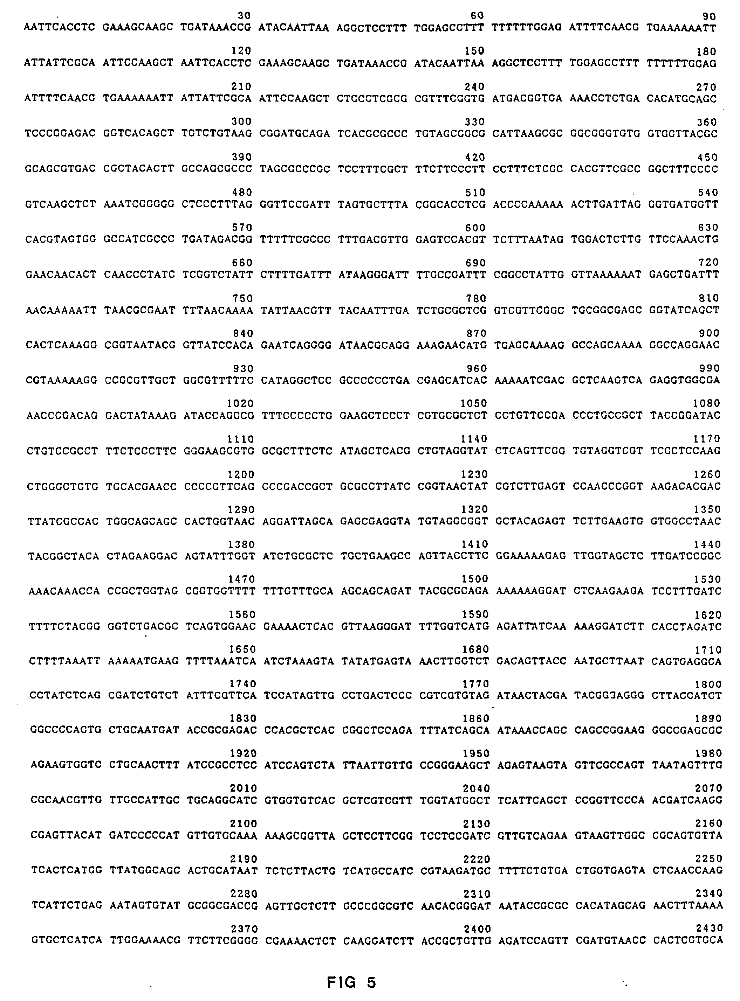

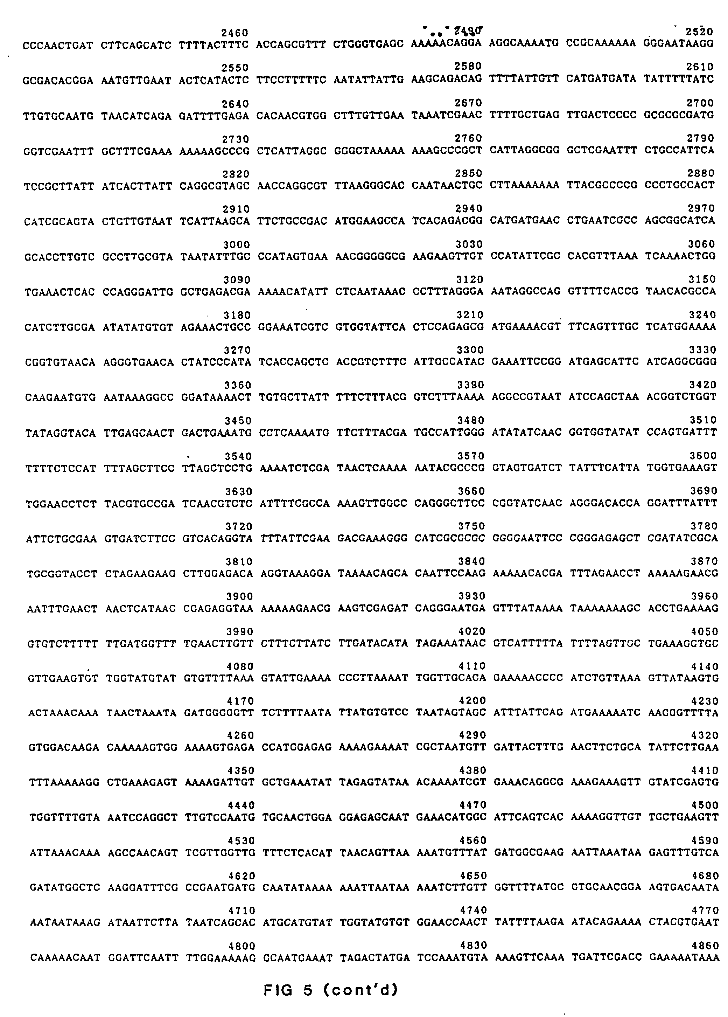

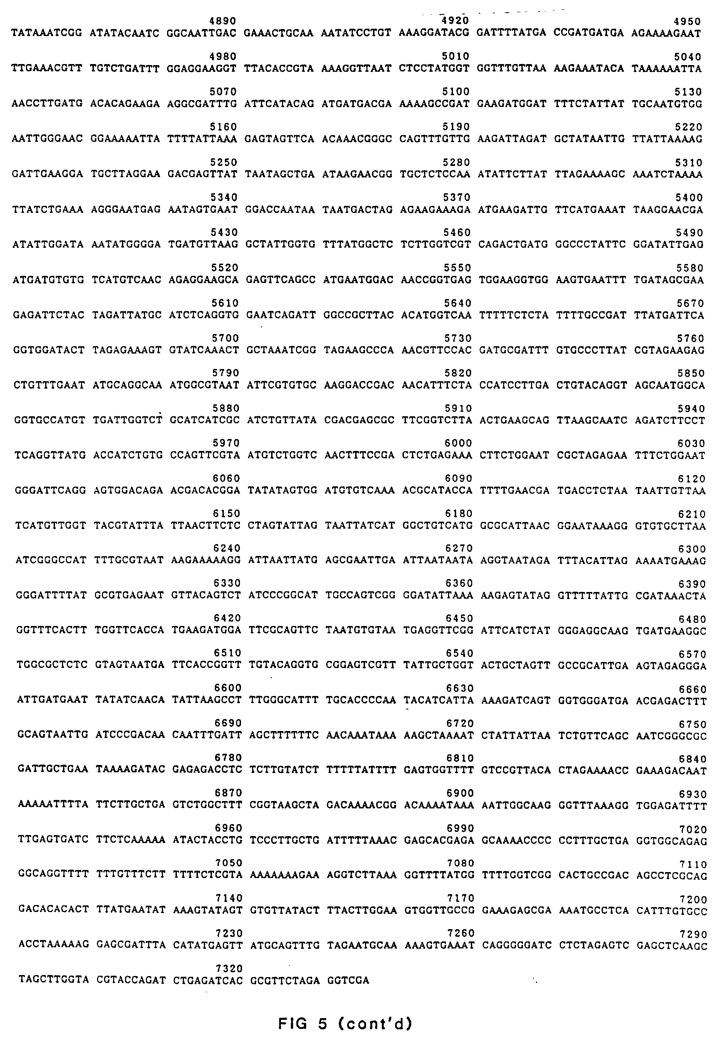

The promoter which may or may not be controllable by regulating its environment. Suitable promoters for prokaryotes include, for example, the trp promoter (inducible by tryptophan deprivation), the lac promoter ( inducible with the galactose analog IPTG), the β-lactamase promoter, and the phage derived P_{L} promoter (inducible by temperature variation).
Additionally, especially for expression in Bacillus, useful promoters include those for alpha-amylase, protease, Spo2, spac and ø105 and synthetic promoter sequences. A preferred promoter is the one depicted in Figure 5 and denoted with "HpaII". Suitable promoters for expression in yeast include the 3-phospho-glycerate kinase promoter and those for other glycolytic enzymes, as well as promoters for alcohol dehydrogenase and yeast phosphatase. Also suited are the promoters for transcription elongation factor (TEF) and lactase. Presently, viral-based insect cell expression systems are also suited, as well as expression systems based on plant cell promoters such as the nopaline synthetase promoters.

Translation control sequences include a ribosome binding site (RBS) in prokaryotic systems, whereas in eukaryotic systems translation may be controlled by a nucleotide sequence containing an initiation codon such as AUG.

In addition to the necessary promoter and the translation control sequences, a variety of other control sequences, including those regulating termination (for example, resulting in polyadenylation sequences in eukaryotic systems) may be used in controlling expression. Some systems contain enhancer elements which are desirable but mostly not obligatory in effecting expression.

A group of vectors denoted with pGBSCC-n, where "n" is any integer from 1 to 17, is especially developed for the DNA encoding the P₄₅₀ SCC enzyme.

Another group of vectors denoted with pGB17α-n, where "n" is any integer from 1 to 5, is especially developed for the DNA encoding the P₄₅₀17α enzyme.

A further group of vectors denoted with pGBC21-n, where "n" is any integer form 1 to 9, is especially developed for the DNA encoding the P₄₅₀C21 enzyme.

Still another group of vectors denoted with pGB11β-n, where "n" is any integer from 1 to 4, is especially developed for the DNA encoding the P₄₅₀11β enzyme.

According to a further aspect of the invention suitable host cells have been selected which accept the vectors of the invention and allow the introduced DNA to be expressed. When culturing the transformed host cells the proteins involved in the conversion of cholesterol to hydrocortisone appear in the cell contents. The presence of the desired DNA can be proved by DNA hybridizing procedures,their transcription by RNA hybridization, their expression by immunological assays and their activity by assessing the presence of oxidized products after incubation with the starting compound in vitro or in vivo.

Transformed microorganisms are preferred hosts, particularly bacteria (more preferably Escherichia coli and Bacillus and Streptomyces species) and yeasts (such as Saccharomyces and Kluyveromyces). Other suitable host organisms are found among plants and non mammalian animals, comprising insects, of which the isolated cells are used in a cell culture, such as Spodoptera frugiperda (Sfg) cell. Alernatively a transgenic plant or non mammalian animal is used.

Recombinant host cells are the ones in which either two or more expression cassettes according to the invention have been introduced or which have been transformed by an expression cassette coding for at least two heterologous proteins, enabling the cell to produce at least two proteins involved in the pathway of figure 1.

A major facture of the invention is that the prepared novel cells are not only able to produce the proteins involved in the oxidative conversion of steroids resulting eventually into hydrocortisone , but also to use these proteins on the spot in the desired oxidative conversion of the corresponding substrate compound added to the culture liquid. Steroids are preferred substrates. The cells transformed with the heterologous DNA are especially suited to be cultured with the steroids mentioned in figure 1, including other sterols such as β-sitosterol. As a result oxidized steroids are obtained.

Depending on the presence in the host cell of a multiplicity of heterologous DNA encoding proteins involved in the pathway of figure 1, several biochemical conversions result comprising the side-chain cleaving of a sterol and/or oxidative modifications on C11, C17, C3 and C21. Therefore the expression cassettes according to the invention are useful in constructing a multigenic system which can effect successive intra-cellular transformations of the multiple steps in the sequence as depicted in figure 1. It may be necessary to introduce into the desired host expression cassettes, which encode in their entirety the required proteins. In some instances, on or more of the proteins involved in the pathway may already be present in the host as a natural protein exerting the same activity. For example, ferredoxin, ferredoxin reductase and P₄₅₀ reductase may already be present in the host. Under those circumstances, only the remaining enzymes must be provided by recombinant transformation.

As an alternative to biochemical conversions in vivo the proteins involved in the conversion of cholesterol to hydrocortisone are collected, purified as far as necessary, and used for the in vitro conversion of steroids in a cell free system, e.g. immobilized on a column. Alternatively the more or less purified mixture containing one or more enzymes of the pathway is used as such for steroid conversion. One exemplified host contains DNA encoding two heterologous proteins viz. the enzyme P₄₅₀SCC and the protein ADX necessary for the production of pregnenolone. In comparison with a host with only P₄₅₀SCC DNA the yield of pregnenolone in a cell-free extract after adding ADR, NADPH and cholesterol is considerably improved.

The present invention provides expression cassettes necessary for the construction of a one-step production process for several useful steroids. Starting from cheap and abundantly available starting compounds, it is especially suited for the production of hydrocortisone and intermediate compounds. The invention renders obsolete traditional expensive chemical reactions. Intermediate compounds need not be isolated. Apart from the novel host cells the processes itself used for culturing these cells on behalf of steroid conversions are analogous to biotechnological procedures well known in the art.

The invention is further illustrated by the following examples which should, however, not be construed as a limitation of the invention

### Example 1

### Molecular cloning of a full-length cDNA encoding the bovine cytochrome P₄₅₀ side chain cleavage enzyme (P₄₅₀SCC)

General cloning techniques as well as DNA and RNA analyses have been used as described in the handbook of T. Maniatis et al., Molecular Cloning, Cold Spring Harbor Laboratory, 1982. Unless described elsewhere all DNA modifying enzymes, molecular cloning vehicles and E.coli strains were obtained from commercial suppliers and used according to the manufacturer's instructions. Materials and apparatus for DNA and RNA separation and purification were used according to instructions of the suppliers.

Bovine adrenal cortex tissue was prepared from freshly obtained bovine kidneys, quickly frozen in liquid nitrogen and stored at -80°C.

From frozen bovine adrenal cortex total cellular RNA was prepared as described by Auffrey and Rougeon (Eur. J. Biochem., 107, 303-314, 1980). Adrenal poly A+ RNA was obtained by heating the total RNA sample at 65°C before polyA selection on oligo(dT) chromatography.

DNA's complementary to polyA⁺ RNA from bovine adrenal cortex were synthesized as follows: 10*µ*g of polyA⁺ RNA, treated with methylmercuric hydroxide was neutralized with beta-mercaptoethanol. This mixture was adjusted to 50 mM Tris/HCl (pH 8.3 at 42°C), 40 mM KCl, 6 mM MgCl₂, 10 mM DTT, 3000 U RNasin/ml, 4 mM Na₄P₂O₇, 50*µ*g actinomycine D/ml, 0.1 mg oligo(dT₁₂₋₁₈)/ml, 0.5 mM dGTP, 0.5 mM dATP, 0.5 mM dTTP, 0.25 mM dCTP and 400 *µ*Ci alpha ³²P-dCTP/ml, all in a final volume of 100 *µ*l. The mixture was put on ice for 10 minutes, heated for 2 minutes at 42°C and the synthesis was started by addition of 150 U AMV reverse transcriptase (Anglian Biotechnology Ltd.); incubation was performed for 1 hr at 42°C.

Second strand synthesis was performed by adding DNA polymerase and RNase H according to Gubler and Hoffman (Gene, 25, 263-269, 1983). After treatment of the ds DNA with T4 DNA polymerase (BRL) to obtain blund ends, decameric EcoRI linkers (Biolabs Inc.) were ligated to the ds DNA fragments. After digestion with EcoRI (Boehringer), double stranded cDNA fragments were separated from the abundant EcoRI-linkers by Biogel A15 m (Bio-Rad) chromatography. Approximately 200 ng EcoRI-linker containing double stranded cDNA was ligated with 10*µ*g of EcoRI digested and calf intestine-phosphatase (Boehringer) treated with lambda-gtll vector DNA (Promega) by T4-DNA ligase (Boehringer) as described by Huynh et al. (In: "DNA cloning techniques: A practical approach", pp. 49-78, Oxford IRL-press, 1985). Phages, obtained after in vitro packaging of the ligation mixture were used to infect the E.coli Y1090 host (Promega).

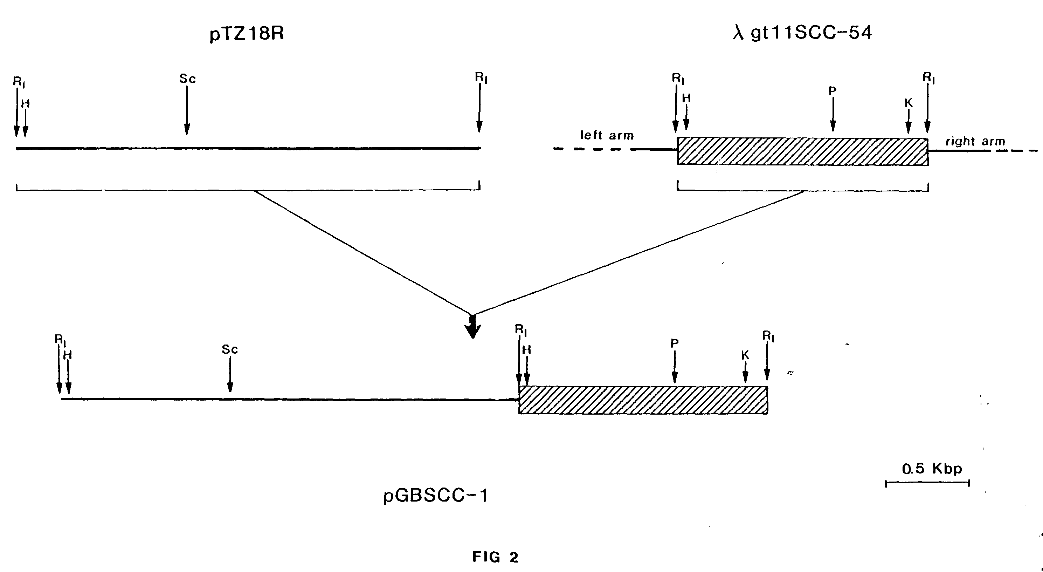

From this cDNA library approximately 10⁶ plaque forming units (pfu's) were screened with a ³²P-end labeled synthetic oligomer SCC-l (5'-GGC TGA CGA AGT CCT GAG ACA CTG GAT TCA GCA CTGG-3'), specific for bovine P₄₅₀SCC DNA sequences as described by Morohashi et al. (Proc. Natl. Acad. Sci. USA, 81, 4647-4651, 1984). Six hybridizing pfu's were obtained and further purified by two additional rounds of infection, plating and hybridization. The P₄₅₀SCCcDNA EcoRI inserts were subcloned into the EcoRI site of pTZ18R (Pharmacia). Clone pGBSCC-1 (figure 2), containing the largest EcoRI insert (1.4 kb), derived from clone lambda-gtll SCC-54 was further analyzed by restriction enzyme mapping and sequencing.

The sequence data revealed that the pGBSCC-1 EcoRI insert was identical with the nucleotide sequence of SCCcDNA between positions 251 and 1824 on the P₄₅₀SCCcDNA map as described by Morohashi et al.

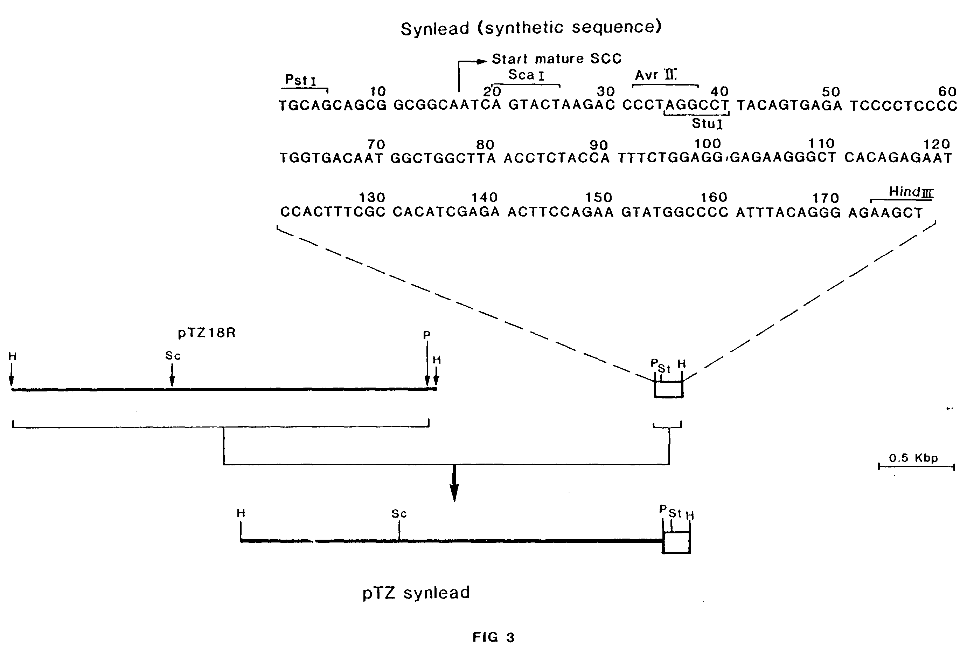

The remaining 5'-P₄₅₀SCCcDNA nucleotides were synthetically derived by cloning a 177 bp Pst/HindIII fragment into the appropriate sites of pTZ18R, resulting in the pTZ/synlead as shown in figure 3, containing besides the nucleotides coding for the mature P₄₅₀SCC protein from position 118 to 273 as published by Morohashi et al., additional restrictive sites for ScaI, AvrII and StuI without affecting the predicted amino acid sequence of the P₄₅₀SCC protein.

The full-length P₄₅₀SCCcDNA was constructed by molecular cloning in E.coli JM101 (ATCC 33876) of a ligation mixture containing the 1372 bp HindIII/KpnI pGBSCC-1 fragment, the 177 bp Pst/HindIII pTZ/synlead fragment and pTZ19R DNA digested with PstI and KpnI.

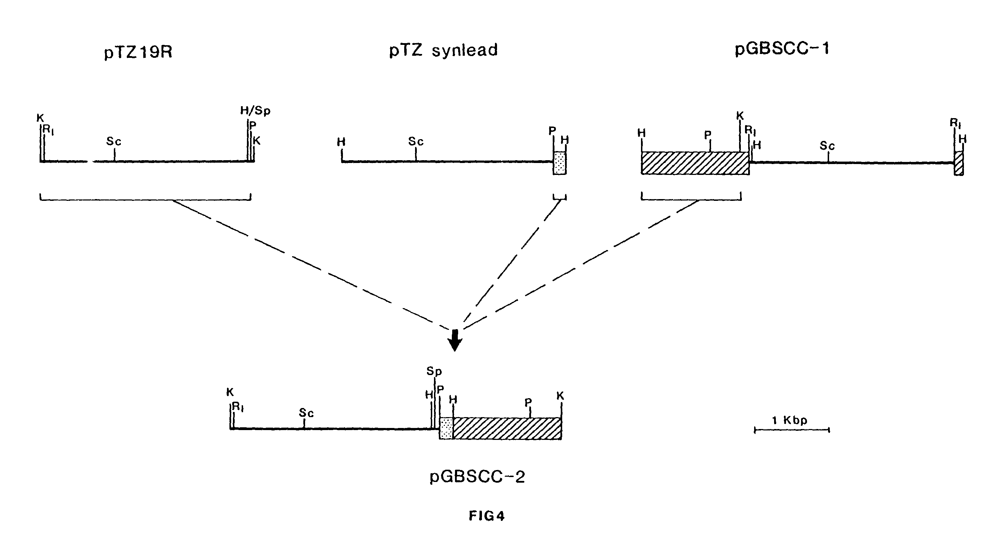

The resulting plasmid, pGBSCC-2, containing all nucleotide sequences encoding the mature bovine P₄₅₀ side chain cleavage protein is shown in figure 4.

### Example 2

### Construction, transformation and expression of P₄₅₀SCC in the bacterial host Bacillus subtilis

To derive expression of cytochrome P₄₅₀SCC in a Bacillus host, P₄₅₀SCCcDNA sequences were transferred to an E.coli/Bacillus shuttle vector pBHA-1.

Figure 5 shows the nucleotide sequence of the shuttle plasmid pBHA-1. The plasmid consists of positions 11-105 and 121-215: bacteriophage FD terminator (double); positions 221-307: a part of plasmid pBR322 (viz. positions 2069-2153); positions 313-768: bacteriophage Fl, origin of replication (viz. positions 5482-5943); positions 772-2571: part of plasmid pBR322, viz. the origin of replication and the β-lactamase gene; positions 2572-2685: transposon TN903, complete genome; positions 2719-2772: tryptophan terminator (double); positions 2773-3729: transposon Tn9, the chloramphenicolacetyltransferase gene. The nucleotides at position 3005 (A), 3038 (C), 3302 (A) and 3409 (A) differ from the wild type cat coding sequence. These mutations were introduced so as to eliminate the NcoI, BalI, EcoRI and PvuII sites: positions 3730-3804: multiple cloning site; positions 3807-7264: part of plasmid pUB110 containing the Bacillus "HpaII" promoter, the replication function and kanamycin resistance gene (EcoRI-PvuII fragment) (McKenzie et al., Plasmid 15, 93-103, 1986 and McKenzie et al., Plasmid 17, 83-85, 1987); positions 7267-7331: multiple cloning site. The fragments were put together by known cloning techniques, e.g. filling in of sticky ends with Klenow, adapter cloning, etc. All data were derived from Genbank^{R}, National Nucleic Acid Sequence Data Bank, NIH, USA.

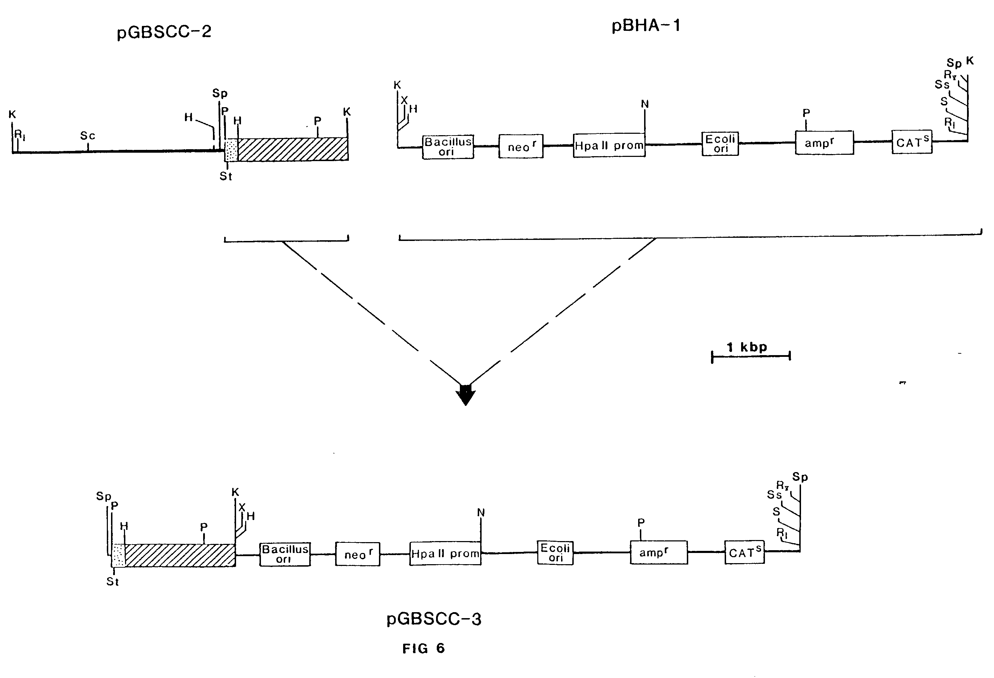

pGBSCC-3 was derived by molecular cloning in E.coli JM101 of the KpnI/SphI P₄₅₀SCCcDNA insert of pGBSCC-2 (described in Example 1) into the appropriate sites in pBHA-1 as indicated in figure 6.

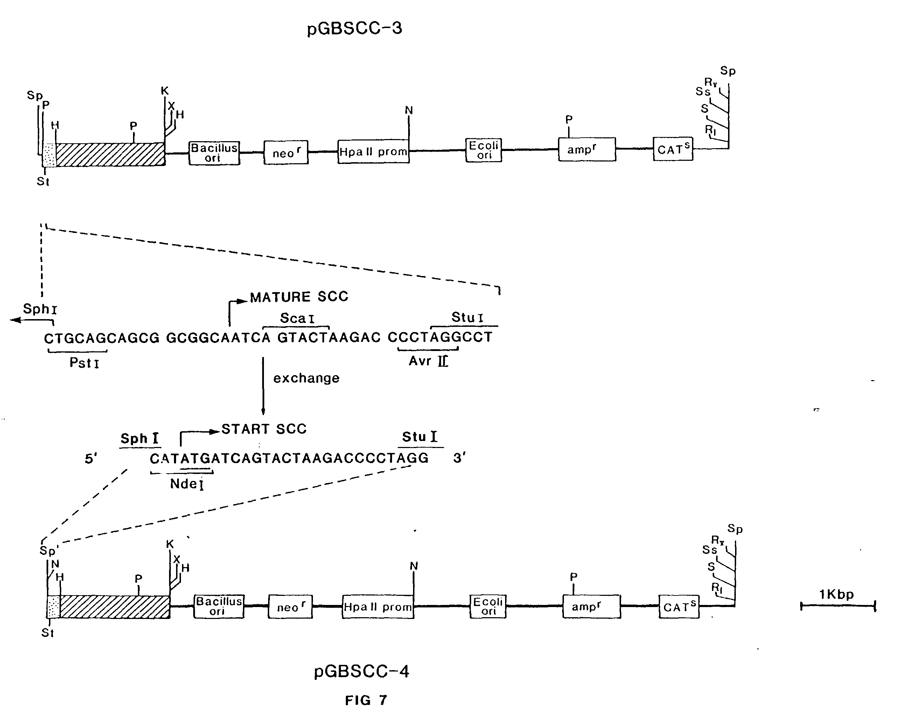

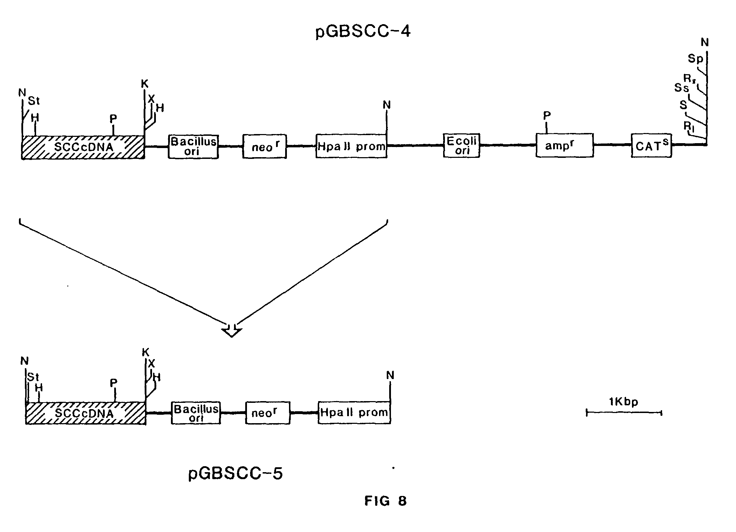

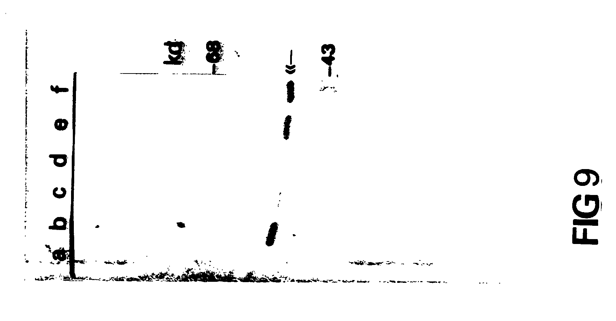

By molecular cloning in E.coli JM101 the methionine initiation codon was introduced by exchanging the StuI/SphI fragment in pGBSCC-3 by a synthetically derived SphI/StuI fragment. 
SPH 1 STU 1
CATATGATCAGTACTAAGACCCCTAGG
GTACGTATACTAGTCATGATTCTGGGGATCC
NDE 1
 containing an NdeI site at the ATG initiation codon. The obtained plasmid pGBSCC-4 is shown in figure 7. The "Hpa II" Bacillus promoter was introduced upstream P₄₅₀SCCcDNA sequences by digestion pGBSCC-4 with the restriction enzyme NdeI, separation of the E.coli part of the shuttle plasmid by agarose gel electrophoresis and subsequent religation and transformation into Bacillus subtilis 1A40 (BGSC 1A40) competent cells. Neomycin resistant colonies were analysed and the plasmid pGBSCC-5 (figure 8) was obtained. Expression of bovine P₄₅₀SCC was studied by preparing a cellular protein fraction of an overnight culture at 37°C in TSB medium (Gibco) containing 10*µ*g/ml neomycin. Cells of 100 *µ*l culture, containing approximately 5.10⁶ cells, were harvested by centrifugation and resuspended in 10 mM Tris/HCl pH 7.5. Lysis was performed by adding lysozym (1 mg/ml) and incubation during 15 minutes at 37°C. After treatment with 0.2 mg DNase/ml during 5 minutes at 37°C the mixture was adjusted to 1x SB buffer, as described by Laemmli, Nature 227, 680-685, 1970, in a final volume of 200 µl. After heating for 5 minutes at 100°C 15 *µ*l of the mixture was subjected to a 7.5% of SDS/ polyacrylamide gel electrophoresis. As shown in figure 9 (lane c) a 53 kDa band could be detected after immunoblotting of the gel probed with P₄₅₀SCC specific antibodies.

Specific bovine P₄₅₀SCC antibodies were obtained by immunisation of rabbits with purified P₄₅₀SCC protein isolated from bovine adrenal cortex tissue.

### Example 3

### Expression of P₄₅₀SCC in the bacterial host Bacillus licheniformis

Expression of bovine P₄₅₀SCC in B.licheniformis was performed by transformation plasmid pGBSCC-5 into the appropriate host strain B.licheniformis T5(CBS 470.83). A cellular protein fraction prepared as described in example 2, from an overnight culture at 37°C in Trypton Soy Broth (TSB) medium (Oxoid) containing 10µg/ml neomycin, was analyzed by SDS/PAGE and Western-blotting. As shown in figure 9 (lane f) a 53 kDa sized protein band was visualised after incubation of the nitrocellulose filter with antibodies specific for bovine P₄₅₀SCC.
One transformant, SCC-201, was further analyzed for in vivo activity of P₄₅₀SCC (see example 11).

### Example 4

### Expression of P₄₅₀SCC in the bacterial host Escherichia coli

### (a) Construction of the expression cassette

To derive a suitable expression vector in the host E.coli for bovine P₄₅₀SCC, pTZ18R was mutated by site-directed mutagenesis as described by Zoller and Smith (Methods in Enzymology 100, 468-500, 1983); Zoller and Smith (Methods in Enzymology 154, 329-350, 1987) and Kramer and Fritz (Methods in Enzymology 154, 350-367, 1987). Plasmids and strains for in vitro mutagenesis experiments were obtained from Pharmacia Inc.

A synthetic derived oligomer with the sequence: 
5'-CAG GAA ACA CAT ATG ACC ATG ATT-3'
NdeI
 was used to create an NdeI restriction site at the ATG initiation codon of the lac Z gene in pTZ18R.

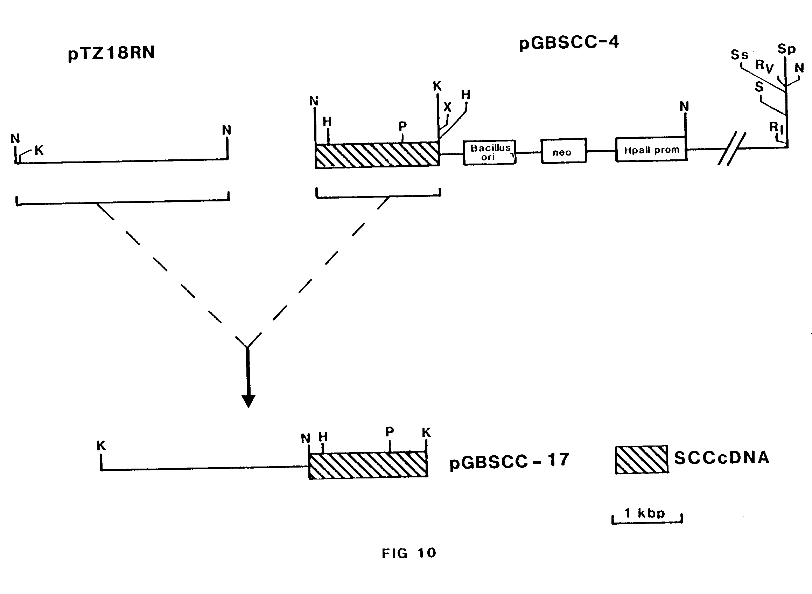

The resulting plasmid pTZ18RN was digested with NdeI and KpnI and the NdeI/KpnI DNA fragment of pGBSCC-4, containing the full-length SCCcDNA, was inserted by molecular cloning as indicated in figure 10.

The transcription of P₄₅₀SCCcDNA sequences in the derived plasmid pGBSCC-17 will be driven by the E.coli lacpromoter.

### (b) Expression of P₄₅₀SCC in the host E.coli JM101

pGBSCC-17 was introduced into E.coli JM101 competent cells by selecting ampicillin resistant colonies. Expression of cytochrome P₄₅₀SCC was studied by preparing a cellular protein fraction (described in example 2) of transformants SCC-301 and 302 from an overnight culture at 37°C in 2xTY medium (containing per liter of de-ionized water: Bacto tryptone (Difco), 16 g; yeast extract (Difco), 10 g and NaCl, 5 g) containing 50 *µ*g/ml ampicillin.

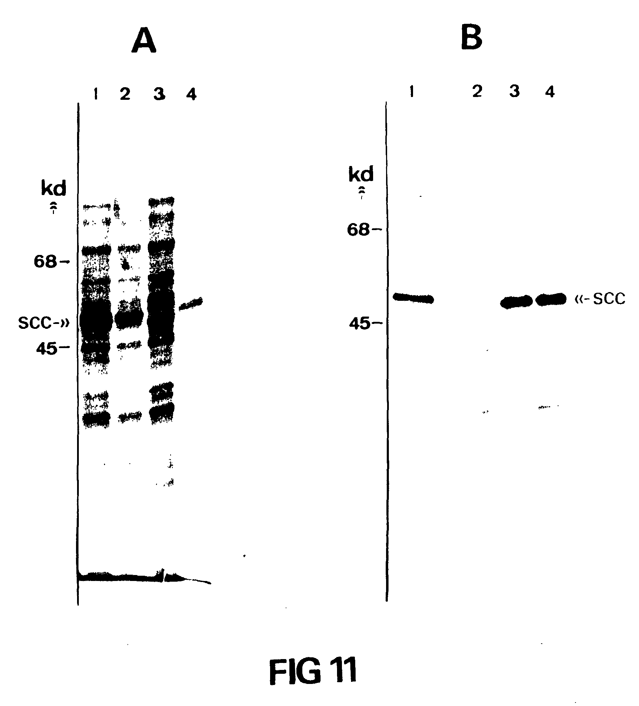

Protein fractions were analyzed by SDS/PAGE stained with Coomassie brilliant blue (figure 11A) or by Western-blot and probed with antibodies specific for bovine P₄₅₀SCC (figure 11B). Both analyses show a protein of the expected length (figure 11A, lanes 1 and 2 and in figure 11B, lanes 3 and 4) for the transformants SCC-301 and SCC-302, resp., which is absent in the E.coli JM101 control strain (figure 11A, lane 3 and figure 11B, lane 2).

### Example 5

### Construction, transformation and expression of P₄₅₀SCC in the yeast Kluyveromyces lactis

### (a) Introduction of the geneticin resistance marker in pUC19

A DNA fragment comprising the Tn5 gene (Reiss et al, EMBO J., 3, 3317-3322, 1984) conferring resistance to geneticin under the direction of the alcohol dehydrogenase I (ADHI) promoter from S.cerevisiae, similar to that described by Bennetzen and Hall (J. Biol. Chem., 257, 3018-3025, 1982) was inserted into SmaI site of pUC19 (Yanisch-Perron et al., Gene, 33, 103-119, 1985). The obtained plasmid pUCG418, is shown in figure 12.

E.coli containing pUCG418 was deposited at Centraal Bureau voor Schimmelcultures under CBS 872.87.

### (b) Construction of the expression cassette

A vector was constructed, comprising pUCG418 (for description see example 5(a)) cut with XbaI and HindIII, the XbaI-SalI fragment from pGB901 containing the lactase promoter (see J.A. van den Berg et al., Continuation-in-part of US patent application serial no. 572.414: Kluyveromyves as a host strain) and synthetic DNA comprising part of the 3' noncoding region of the lactase gene of K.lactis. This plasmid, pGB950, is depicted in figure 13. pGB950 was cut with SalI and XhoI and synthetic DNA was inserted: 
SAL 1 STU 1 XHO 1
TCGACAAAAATGATCAGTACTAAGACTCCTAGGCCTATCGATTC
GTTTTTACTAGTCATGATTCTGAGGATCCGGATAGCTAAGAGCT
 resulting in plasmid pGBSCC-6 as shown in figure 13.

The StuI-EcoRI fragment from pGBSCC-2 (see example 1) containing the P₄₅₀SCC coding region was isolated and the sticky end was filled in, using Klenow DNA polymerase. This fragment was inserted into pGBSCC-6 cut with StuI. The plasmid containing the fragment in the correct orientation was called pGBSCC-7 (see figure 14).

### (c) Transformation of K.lactis

K.lactis strain CBS 2360 was grown in 100 ml of YEPD-medium (1% yeast extract, 2% peptone, 2% glucosemonohydrate) containing 2.5 ml of a 6.7% (w/w) yeast nitrogen base (Difco laboratories) solution to an OD₆₁₀ of about 7. From 10 ml of the culture the cells were collected by centrifugation, washed with TE-buffer (10 mM Tris-HCl pH 7.5; 0.1 mM EDTA) and resuspended in 1 ml TE-buffer. An equal volume of 0.2 M lithium acetate was added and the mixture was incubated for 1 hr at 30°C in a shaking waterbath. 15*µ*g of pGBSCC-7 was cut at the unique SacII site in the lactase promoter, ethanol precipitated and resuspended in 15 *µ*l TE-buffer. This DNA preparation was added to 100 *µ*l of the pre-incubated cells and the incubation was prolonged for 30 minutes. Then an equal volume of 70% PEG4000 was added and the mixture was incubated for 1 hr at the same temperature, followed by a heatshock of 5 minutes at 42°C. Then 1 ml of YEPD-medium was added and the cells were incubated for 1.5 hrs in a shaking waterbath of 30°C. Finally the cells were collected by centrifugation, resuspended in 300 *µ*l YEPD and spread on agar plates containing 15 ml of YEPD agar with 300*µ*g/ml of geneticin and were overlayered 1 hr before use with 15 ml YEPD-agar without G418. Colonies were grown for 3 days at 30°C.

### (d) Analysis of the transformants

Transformants and the control strain CBS 2360 were grown in YEPD medium for about 64 hrs at 30°C. The cells were collected by centrifugation, resuspended in a physiological salt solution at an OD₆₁₀ of 300 and disrupted by shaking with glass beads for 3 minutes on a Vortex shaker at maximum speed. Cell debris was removed by centrifugation for 10 minutes at 4500 rpm in a Hearaeus Christ minifuge GL. From the supernatants 40 *µ*l samples were taken for analysis on immunoblots (see figure 15A, lane 3 and figure 15B, lane 4).

The results show that a protein of the expected length is expressed in K.lactis cells transformed with pGBSCC-7. The transforman was denoted as K.lactis SCC-101.

### Example 6

### Construction, transformation and expression of P₄₅₀SCC in the yeast Saccharomyces cerevisiae

### (a) Construction of the expression cassette

In order to delete the lactase promoter, pGB950 (see example 4(b)) was cut with XbaI and SalI, the sticky ends were filled in using Klenow DNA polymerase and subsequently ligated. In the resulting plasmid, pGBSCC-8, the XbaI-site is destroyed, but the SalI site is maintained.

The SalI-fragment from pGB161 (see J.A. van den Berg et al., EP 96430) containing the isocytochrome CI (cyc 1) promoter from S.cerevisiae was isolated and partially digested with XhoI. The 670 bp XhoI-SalI fragment was isolated and cloned into the SalI-site of pGBSCC-8. In the selected plasmid, pGBSCC-9, the SalI-site between the cyc 1 promoter and the 3' noncoding region of the lactase gene is maintained (figure 16) (HindIII partially digested).

The SalI-HindIII fragment from pGBSCC-7, containing the P₄₅₀SCC coding region was inserted in pGBSCC-9 cut with SalI and HindIII. In the resulting plasmid, pGBSCC-10, the P₄₅₀SCC coding region is downstream to the cyc 1 promoter (figure 17).

### (b) Transformation of S.cerevisiae

S.cerevisiae strain D273-10B (ATCC 25657) was grown in 100 ml YEPD overnight at 30°C, subsequently diluted (1:10000) in fresh medium and grown to an OD₆₁₀ of 6. The cells from 10 ml of the culture were collected by centrifugation and suspended in 5 ml TE-buffer. Again the cells were collected by centrifugation, suspended in 1 ml of the TE-buffer and 1 ml 0.2 M lithium acetate was added. The cells were incubated for 1 hour in a shaking waterbath at 30°C. 15 µg pGBSCC-10 was cut at the unique MluI-site in the cyc 1 promoter, ethanol precipitated and resuspended in 15 µl TE. This DNA preparation was added to 100 *µ*l of the pre-incubated yeast cells and incubated (shaking) for 30 minutes at 30°C. After addition of 115 *µ*l of a 70% PEG4000 solution the incubation was prolonged 60 minutes, without shaking. Subsequently a heat shock of 5 minutes at 42°C was given to the cells, 1 ml YEPD medium was added, followed by a 1½ hour incubation at 30°C in a shaking waterbath. Finally the cells were collected by centrifugation, resuspended in 300 *µ*l YEPD and spread on YEPD agar plates containing geneticin (300 µg/ml).
Colonies were grown for three days at 30°C.

### (c) Analysis of the transformants

Transformants and the control strain were grown in YEPL-medium (1% yeast extract, 2% bactopeptone, 3.48% K₂HPO₄ and 2.2% of a 90% L-(+)-lactic acid solution; before sterilization the pH was adjusted to 6.0 using a 25% ammonia solution) for 64 hrs at 30°C. Further analysis was done as described in example 5(d).

The immunoblot-analysis demonstrates the expression of P₄₅₀SCC in S.cerevisiae (figure 15A, lane 1).

### Example 7

### Construction, transformation and expression of pre-P₄₅₀SCC encoding DNA in the yeast Kluyveromyces lactis

### (a) Construction of the expression cassette

Plasmid pGB950 (see example 5(b)) was cut with SalI and XhoI and synthetic DNA was inserted: 
SAL I
TCGACAAAAATGTTGGCTCGAGGTTTGCCATTGAGATCCGCTTTGGTTAAGGCTTGTCC
GTTTTTACAACCGAGCTCCAAACGGTAACTCTAGGCGAAACCAATTCCGAACAGG

ACCAATCTTGTCCACTGTTGGTGAAGGTTGGGGTCACCACAGAGTTGGTACTGGTGAAGG
TGGTTAGAACAGGTGACAACCACTTCCAACCCCAGTGGTGTCTCAACCATGACCACTTCC

STU 1 XHO 1
TGCTGGTATCAGTACTAAGACTCCTAGGCCTATCGATTC
ACGACCATAGTCATGATTCTGAGGATCCGGATAGCTAAGAGCT
 resulting in plasmid pGBSCC-11 (figure 18). Analogous as described in example 5(b), the P₄₅₀SCC coding region of pGBSCC-2 was inserted into pGBSCC-11 cut with StuI. The plasmid containing the fragment in the correct orientation was called pGBSCC-12 (figure 18).

### (b) Transformation of K.lactis and analysis of the transformants

Transformation of K.lactis with pGBSCC-12 was performed as described in example 5(c). The transformants were analysed as described in example 5(d). The analysis demonstrates the production of P₄₅₀SCC by K.lactis (figure 15B, lane 3).

### Example 8

### Construction. transformation and expression of pre-P₄₅₀SCC encoding DNA in the yeast Saccharomyces cerevisiae

### (a) Construction of the expression cassette

The SalI-HindIII (HindIII partially digested) fragment from pGBSCC-12, containing the pre-P₄₅₀SCC coding region was inserted in pGBSCC-9 cut with SalI and HindIII. The resulting plasmid was called pGBSCC-13 (figure 19).

### (b) Transformation of S.cerevisiae and analysis of the transformants

S.cerevisiae strain D273-10B was transformed with pGBSCC-13 as described in example 6(b). The transformants were analysed as described in example 5(c). The result, shown in figure 15C (lane 3), demonstrates the expression of P₄₅₀SCC by S.cerevisiae. One transformant, SCC-105, was further analyzed for in vitro activity of P₄₅₀SCC (see example 12).

### Example 9

### Construction, transformation and expression in Kluyveromyces lactis of P₄₅₀SCC sequences fused to the pre-region of cytochrome oxidase VI from Saccharomyces cerevisiae

### (a) Construction of the expression cassette

Plasmid pGB950 (see example 6(b)) was cut with SalI and XhoI and synthetic DNA was inserted: 
SAL I
TCGACAAAAATGTTGTCTCGAGCTATCTTCAGAAACCCAGTTATCAACAGAACTTTGTT
GTTTTTACAACAGAGCTCGATAGAAGTCTTTGGGTCAATAGTTGTCTTGAAACAA

GAGAGCTAGACCAGGTGCTTACCACGCTACTAGATTGACTAAGAACACTTTCATCCAATC
CTCTCGATCTCGTCCACGAATGGTGCGATGATCTAACTGATTCTTGTGAAAGTAGGTTAG

STU 1 XHO 1
CAGAAAGTACATCAGTACTAAGACTCCTAGGCCTATCGATTC
GTCTTTCATGTAGTCATGATTCTGAGGATCCGGATAGCTAAGAGCT
 resulting in plasmid pGBSCC-14.

The amino acid sequence from the cytochrome oxidase VI (COX VI) pre-sequence was taken from the article of Wright et al. (J. Biol. Chem., 259, 15401-15407, 1984). The synthetic DNA was designed, using preferred yeast codons. The P₄₅₀SCC coding region of pGBSCC-2 was inserted into pGBSCC-14 cut with StuI, similarly as described in example 5(b). The plasmid containing the P₄₅₀SCC coding sequence in frame with the COX VI pre-sequence was called pGBSCC-15 (figure 20).

### (b) Transformation of K.lactis and analysis of the transformants

Transformation of K.lactis with pGBSCC-15 was performed as described in example 5(c). The transformants were analysed as described in example 5(d). The result (figure 15B, lane 2) shows that P₄₅₀SCC is expressed.

### Example 10

### Construction, transformation and expression in Saccharomyes cerevisiae of P₄₅₀SCC sequences fused to the pre-region of cytochrome oxidase VI from Saccharomyces cerevisiae

### (a) Construction of the expression cassette

The SalI-HindIII (HindIII partially digested) fragment from pGBSCC-15, containing the coding region for P₄₅₀SCC fused to the COX VI pre-sequence, was inserted in pGBSCC-9 cut with SalI and HindIII. The resulting plasmid was called pGBSCC-16 (figure 21).

### (b) Transformation of S.cerevisiae and analysis of the transformants

S.cerevisiae strain D273-10B was transformed with pGBSCC-16 as described in example 6(b). The transformants were analysed as described in example 6(c). The result, shown infigure 15C (lane 2), demonstrates the expression of P₄₅₀SCC in S.cerevisiae.

### Example 11

### In vivo activity of P₄₅₀SCC in Bacillus licheniformis SCC-201

B.licheniformis SCC-201 was obtained as described in example 3. The organism was inoculated in 100 ml of medium
A. Medium A consisted of:

| | |
|---|---|
| Calcium chloride-hexahydrate | 1 g |
| Ammonium sulfate | 5 g |
| Magnesium chloride-hexahydrate | 2.25 g |
| Manganese sulfate-tetrahydrate | 20 mg |
| Cobalt chloride-hexahydrate | 1 mg |
| Citric acid-monohydrate | 1.65 g |
| Distilled water | 600 ml |
| Trace elements stock solution | 1 ml |
| Antifoam (SAG 5693) | 0.5 mg |

Trace elements stock solution contained per l of distilled water:

| | |
|---|---|
| CuSO₄.5H₂O | 0.75 g |
| H₃BO₃ | 0.60 g |
| KI | 0.30 g |
| FeSO₄(NH₄)₂SO₄.2H₂O | 27 g |
| ZnSO₄.7H₂O | 5 g |
| Citric acid.H₂O | 15 g |
| MnSO₄.H₂O | 0.45 g |
| Na₂MoO₄.H₂O | 0.60 g |
| H₂SO₄ (96%) | 3 ml |

After sterilisation and cooling to 30°C in order to complete the medium, 60 g of maltose-monohydrate dissolved in 200 ml of distilled water (sterilized 20 minutes, 120°C), 200 ml 1M of potassium phosphate buffer (pH 6.8; sterilized 20 minutes, 120°C), 1.7 g of Yeast Nitrogen base (Difco) dissolved in 100 ml of distilled water (sterilized by membrane filtration) were added to the medium.

The culture was grown for 64 hours at 37°C and subsequently 2 ml of this culture was added as inoculum to 100 ml of medium A containing 10 mg of cholesterol. Cholesterol was added as a solution containing cholesterol 10 mg; Tergitol™/ethanol (1:1, v/v), 0.75 ml and Tween 80™, 20 *µ*l. The culture was grown for 48 hours at 37°C, whereupon the culture was extracted with 100 ml of dichloromethane. The mixture was separated by centrifugation and the organic solvent layer was collected. The extraction procedure was repeated twice and the 3 x 100 ml of dichloromethane fractions were pooled. The dichloromethane was evaporated by vacuum distillation and the dried extract (approximately 450 mg) was analysed for pregnenolone using a gaschromatograph-mass spectrometer combination.

### GC-MS analysis.

From the dried extract a defined amount was taken and silylated by adding a mixture of pyridine bis-(trimethylsilyl)-trifluoroacetamide and trimethylchlorosilane. The silylated sample was analysed by a GL-MS-DS combination (Carlo Erba MEGA 5160-Finnigan MAT 311A-Kratos DS 90) in the selected ion mode. Gaschromatography was performed under the following conditions: injection moving needle at 300°C; column M.cpsil29 0.25 inner diameter df 0.2 *µ*m operated at 300°C isotherm; direct introduction into MS-source.

Samples were analysed by monitoring ions m/z 298 from pregnenolone at a resolution of 800. From the measurements it is clear that in case of the host strain B.licheniformis T5 no pregnenolone could be detected (detection limit 1 picogram), whereas in case of B.licheniformis SCC-201 production of pregnenolone easily could be monitored.

### Example 12

### In vitro activity of P₄₅₀SCC obtained from Saccharomyces cerevisiae SCC-105

S.cerevisiae SCC-105 obtained as described in example 8 was inoculated in 100 ml medium B. Medium B contained per l of distilled water:

| | |
|---|---|
| Yeast extract | 10 g |
| Bacto Peptone (Oxoid) | 20 g |
| Lactic acid (90%) | 20 g |
| Dipotassium phosphate | 35 g |
| pH = 5.5 (adjusted with ammonia, 25% w/w) | |

This culture was grown for 48 hours at 30°C and subsequently this culture was used as inoculum for a fermentor containing medium C. Medium C consisted of:

| | |
|---|---|
| Yeast extract | 100 g |
| Bacto Peptone (Oxoid) | 200 g |
| Lactic acid (90%) | 220 ml |
| Dipotassium hydrogen phosphate | 35 g |
| Distilled water | 7800 ml |
| pH was adjusted at pH = 6.0 with ammonia (25%) and the fermentor including the medium was sterilized (1 hour, 120°C). | |

After cooling, 2.4 g of geneticin dissolved in 25 ml of distilled water was sterilized by membrane filtration and added to the medium. The inoculated mixture was grown in the stirred reactor (800 rpm) at 30°C, while sterile air was passed through the broth at a rate of 300 l/h and the pH was automatically kept at 6.0 with 4N H₂SO₄ and 5% NH₄OH (5% NH₄OH in distilled water; sterilized by membrane filtration). After 48 hours a feed of lactic acid (90%, sterilized by membrane filtration) was started at a rate of 20 g/h. The fermentation is then resumed for 40 hours, whereupon the cells were collected by centrifugation (4000xg, 15 minutes).
The pellet was washed with 0.9% (w/w) NaCl, followed by centrifugation (4000xg, 15 minutes); the pellet washed with phosphate buffer (50 mM, pH = 7.0) and cells were collected by centrifugation (4000xg, 15 minutes). The pellet was taken up in phosphate buffer (50 mM, pH = 7.0) resulting in a suspension containing 0.5 g wet weight/ml. This suspension was treated in a Dyno^{R}-mill (Willy A. Bachofen Maschinenfabrik, Basel, Schweiz). Unbroken cells were removed by centrifugation (4000xg, 15 minutes). The cell-free extract (2250 ml, 15-20 mg protein/ml) was stored at -20°C.

P₄₅₀SCC was roughly purified by the following procedure. From 50 ml of thawed cell-free extract, a rough membrane fraction was pelleted by ultracentrifugation (125000xg, 30 minutes) and resuspended in 50 ml of a 75 mM potassium phosphate solution (pH 7.0), containing 1% of sodium cholate. This dispersion was gently stirred for 1 hour at 0°C, and subsequently centrifugated (125000xg, 60 minutes). To the thus obtained supernatant, containing solubilized membrane proteins, (NH₄)₂SO₄ was added (30% w/v), while the pH was kept at 7.0 by adding small amounts of a NH₄OH solution (6N). The suspension was stirred for 20 minutes at 0°C, after which the fraction of precipitated proteins was collected by centrifugation (15000xg, 10 min). The pellet was resuspended to 2.5 ml with 100 mM potassium phosphate buffer (pH 7.0), containing 0.1 mM dithio-threitol and 0.1 mM EDTA. This suspension was eluted over a gelfiltration column (PD10, Pharmacia), yielding 3.5 ml of a desalted protein fraction (6 mg/ml), which was assayed for P₄₅₀SCC activity.

P₄₅₀SCC activity was determined by an assay, which is essentially based on a method of Doering (Methods Enzymology, 15, 591-596, 1969). The assay mixture consisted of the following solutions:
Solution A (natural P₄₅₀SCC electron donating system):
   a 10 mM potassium phosphate buffer (pH 7.0), containing 3 mM of EDTA, 3 mM of phenylmethylsulfonyl fluoride (PMSF), 20 *µ*M of adrenodoxin and 1 *µ*M of adrenodoxin reductase (electron carriers; both purified from bovine adrenal cortex),
   1 mM of NADPH (electron donor) and
   15 mM glucose-6-phosphate and
   8 units/ml glucose-6-phosphate-dehydrogenase (NADPH regenerating system).
Solution B (substrate): a micellar solution of 37.5 *µ*M cholesterol (doubly radiolabeled with [26,27-¹⁴C] cholesterol (40 Ci/mol) and [7 alpha-³H] cholesterol (400 Ci/mol)) in 10% (v/v) Tergitol™ NP40/ethanol (1:1, v/v).

The assay was started by mixing 75 *µ*l of solution A with 50 *µ*l of solution B and 125 *µ*l of the roughly purified P₄₅₀SCC fraction (or buffer as reference). The mixture was stirred gently at 30°C. Samples (50 *µ*l) were drawn after 0, 30 and 180 minutes and diluted with 100 *µ*l of water. Methanol (100 µl) and chloroform (150*µ*l) were added to the diluted sample. After extraction and centrifugation (5000xg, 2 minutes) the chloroform layer was collected and dried. The dry residue was dissolved in 50 *µ* l of acetone, containing 0.5 mg of a steroid mixture (cholesterol, pregnenolone and progesterone (1:1:1, w/w/w)) and subsequently 110 µl of concentrated formic acid was added. The suspension was heated for 15 minutes at 120°C. Hereafter the ¹⁴C/³H ratio was determined by double label liquid scintillation counting. This ratio is a direct measure for the sidechain cleavage reaction, because the ¹⁴C-labeled sidechain is evaporated from the mixture as isocaprylic acid during the heating procedure.

Using this assay it was found that the P₄₅₀SCC fraction, roughly purified from S.cerevisiae SCC-105, showed side chain cleavage acitivity. During 3 hours of incubation 45% of the cholesterol had been converted. By means of thin layer chromatography the reaction product was identified as pregnenolone.

### Example 13

### Molecular cloning of a full-length cDNA encoding the bovine cytochrome P₄₅₀ steroid 17α-hydroxylase (P₄₅₀17α) .

Approximately 10⁶ pfu's of the bovine adrenal cortex cDNA library described in example 1 was selected for P₄₅₀17αcDNA sequences by screening with two ³²P-end labeled synthetic oligomers specific for P₄₅₀17αcDNA. Oligomer 17α-1 (5'-AGT GGC CAC TTT GGG ACG CCC AGA GAA TTC-3') and oligomer 17α-2 (5'-GAG GCT CCT GGG GTA CTT GGC ACC AGA GTG CTT GGT-3') are complementary to the bovine P₄₅₀17αcDNA sequence as described by Zuber et al. (J. Biol. Chem., 261, 2475-2482, 1986) from position 349 to 320 and 139 to 104, respectively.

Selection with oligomer 17α-1 revealed ± 1500 hybridizing pfu's. Several hybridizing pfu's were selected, purified and scaled up for preparative phage DNA isolation. The EcoRI inserts of the recombinant lambda-gtll DNA's were subcloned in the EcoRI site of pTZ18R. One clone, pGB17α-1, was further characterized by restriction endonuclease mapping and DNA-sequencing. Plasmid pGB17α-1 contains an 1.4 kb EcoRI insert complementary to the 3' part of P₄₅₀17α from the EcoRI site at position 320 to the polyadenylation site at position 1721 as described by Zuber et al. A map of pGB17α-1 is shown in figure 22A.

Eight hybridizing pfu's were obtained by selecting the cDNA library with oligomer 17α-2. After purification, upscaling of recombinant phages and isolation of rec lambda-gtll DNA's, EcoRI inserts were subcloned in the EcoRI site of pTZ18R. EcoRI inserts varied in length from 270 bp to 1.5 kbp. Only one clone, pGB17α-2 containing a 345 bp EcoRI-fragment was further investigated by nucleotide suquencing and compared with the published P₄₅₀17αcDNA sequence data by Zuber et al. As shown in figure 22B the P₄₅₀17αcDNA sequence in pGB17α-2 starts 72 bp upstream the predicted AUG start codon at position 47 and shows complete homology with the 5' part of P₄₅₀17αcDNA till the EcoRI site at position 320 as described by Zuber et al.

A full-length bovine P₄₅₀17αcDNA was constructed by molecular cloning in E.coli JM101 of a ligation mixture containing a partial EcoRI digest of pGB17α-1 and the 345 bp EcoRI fragment of pGB17α-2. The obtained clone pGB17α-3 contains a full-length bovine P₄₅₀17αcDNA and is shown in figure 22C.

### Example 14

### Construction and transformation of a full-length P₄₅₀17αc-DNA clone into the yeast Kluyveromyces lactis

### (a) Construction of the expression vector

To derive a suitable expression vector in yeast hosts for bovine P₄₅₀17α, pGB17α-3 was mutated by site-directed mutagenesis as described by Zoller and Smith, (Methods in Enzymol., 100, 468-500, 1983); Zoller and Smith, (Methods in Enzymol., 154, 329-350, 1987) and Kramer and Fritz, (Methods in Enzymol., 154, 350-367, 1987). Plasmids and strains for in vitro mutagenesis experiments were obtained from Pharmacia Inc..

As indicated in figure 23, 9 bp just upstream the ATG initiation codon were changed to obtain a SalI restriction site and optimal yeast translation signals using the synthetic oligomer 17α-3

SAL 1
5'-TCTTTGTCCTGACTGCTGCCAGTCGACAAAAATGTGGCTGCTC-3'

The resulting plasmid pGB17α-4 was digested with SalI and SmaI; the DNA-fragment containing the full length P₄₅₀17αcDNA was separated by gelectrophoresis, isolated and transferred by molecular cloning in E.coli JM101 into the pGB950 vector (see example 5) which was first digested with XhoI, sticky ends filled in with Klenow DNA polymerase and subsequently digested with SalI, resulting in the plasmid pGB17α-5 as depicted in figure 24.

### (b) Transformation of K.lactis

15 *µ*g of pGb17α-5, cut at the unique SacII site in the lactase promoter, was used to transform K.lactis strain CBS 2360 as indicated in example 5. Transformants were analyzed for the presence of integrated pGB17α-5 sequences in the host genome by southern analysis. One transformant 17α-101, containing at least three copies of pGB17α-5 in the genomic host DNA, was further analyzed for in vivo activity of P₄₅₀17α (see example 16).

### Example 15

### Construction and transformation of P₄₅₀17α in the bacterial hosts Bacillus subtilis and Bacillus licheniformis

### (a) Construction of the expression vector

To derive a suitable expression vector in Bacillus hosts for bovine P₄₅₀17α, pGB17α-3 was mutated by site-directed mutagenesis as described in example 14.

As indicated in figure 25 an NdeI restriction site was introduced at the ATG initation codon using the synthetic oligomer 17α-4:

5'-GCT GCC ACC CAG ACC ATA TGT GGC TGC TCC T-3'
NdeI

The resulting plasmid pGB 17α-6 was partial digested with EcoRI: the DNA fragment containing the full-length P₄₅₀17αcDNA was separated by gelelectrophoresis, isolated and ligated to EcoRI digested pBHA-1 DNA as shown in figure 26. The ligate was molecular cloned by transferring the ligation mixture into E.coli JM101 to obtain pGB17α-7.

### (b) Transformation of B.subtilis and B.licheniformis

The "HpaII" Bacillus promoter was introduced upstream the P₄₅₀17αcDNA sequences by digestion pGB17α-6 with the restriction enzyme NdeI, separation of the E.coli part of the shuttle plasmid by agarose gel electrophoresis and subsequent religation and transformation of B.subtilis 1A40 (BGSC 1A40) competent cells. Neomycin resistant colonies were analysed and the plasmid pGB17α-8 (figure 27) was obtained.

Transformation of the host B.licheniformis T5 (CBS 470.83) was also performed with pGB17α-8. The plasmid remains stable in the appropriate Bacillus hosts as revealed by restriction analysis of pGB17α-8 even after many generations.

### Example 16

### In vivo activity of P₄₅₀17α in Kluyveromyces lactis 17α-101

K.lactis 17α-101 was obtained as described in example 14. The organism was inoculated in 100 ml of medium D. Medium D contained per litre of distilled water:

| | |
|---|---|
| Yeast Extract (Difco) | 10 g |
| Bacto Peptone (Oxoid) | 20 g |
| Dextrose | 20 g |

After sterilization and cooling to 30°C, 2.68 g of Yeast Nitrogen Base (Difco) dissolved in 40 ml of distilled water (sterilized by membrane filtration) and 50 mg of neomycine dissolved in 1 ml of distilled water (sterilized by membrane filtration) was added to the medium. Subsequently 50 mg of progesterone dissolved in 1.5 ml dimethylformamide was added to 100 ml of medium. The culture was grown for 120 hours at 30°C and subsequently 50 ml of culture broth was extracted with 50 ml of dichloromethane. The mixture was centrifugated and the organic solvent layer was separated. Dichloromethane was evaporated by vacuum distillation and the dried extract (about 200 mg) was taken up in 0.5 ml of chloroform. This extract contained 17α-hydroxyprogesterone as shown by thin layer chromatography. The structure of the compound was confirmed by H-NMR and ¹³C-NMR. NMR analysis also showed that the ratio 17α-hydroxyprogesterone/progesterone in the extract was approximately 0.3.

### Example 17

### Molecular cloning of a full-length cDNA encoding the bovine cytochrome P₄₅₀ steroid 21-hydroxylase (P₄₅₀C21)

Approximately 10⁶ Pfu's of the bovine adrenal cortex cDNA library, prepared as described in example 1, were hybridized with a ³²P-end labeled oligo C21-1. This oligo, containing the sequence 5'- GAT GAT GCT GCA GGT AAG CAG AGA GAA TTC-3' is a specific probe for the bovine P₄₅₀C21 gene located downstream the EcoRI site in the P₄₅₀C21 cDNA sequence as described by Yoshioka et al. (J. Biol. Chem., 261, 4106-4109, 1986). From the screening one hybridizing pfu was obtained. The EcoRI insert of this recombinant lambda-gt11 DNA was subcloned in the EcoRI site of pTZ18R resulting in a construct called pGBC21-1. As shown in figure 28 this plasmid contains a 1.53 kb EcoRI insert complementary to the P₄₅₀C21cDNA sequences from the EcoRI site at position 489 to the polyadenylation site as described by Yoshioka et al., as revealed by nucleotide sequencing.

To isolate the remaining 5' part (490 bp) of the P₄₅₀C21cDNA, a new bovine adrenal cortex cDNA Library was prepared according the procedure as described in example 1 with only one modification. As primer for the first cDNA strand synthesis an additional oligomer C21-2 was added. Oligomer C21-2 with the nucleotide sequence 5'- AAG CAG AGA GAA TTC-3' is positioned downstream the EcoRI-site of P₄₅₀C21cDNA from position 504 to 490.

Screening of this cDNA library with a ³²P-end labeled oligomer C21-3, containing the P₄₅₀C21 specific sequence 5'-CTT CCA CCG GCC CGA TAG CAG GTG AGC GCC ACT GAG-3' (positions 72 to 37) revealed approximately 100 hybridizing pfu's. The EcoRI-insert of only one recombinant lambda-gt11 DNA was subcloned in the EcoRI-site of pTZ18R resulting in a construct called pGBC21-2. This plasmid (figure 28) contains an insert of 540 bp complementary to the P₄₅₀C21cDNA sequences from position -50 to the EcoRI-site at position 489 as revealed by nucleotide sequencing.

### Example 18

### Construction of a P₄₅₀C21cDNA Bacillus expression vector and transformation to the bacterial hosts Bacillus subtilis and Bacillus licheniformis

### (a) Construction of the expression vector

To construct a full-length P₄₅₀C21cDNA with flanking sequences specific for the Bacillus expression vector pBHA-1, the 5' part of the P₄₅₀C21 gene was first modified by the Polymerase Chain Reaction (PCR) method with pGBC21-2 as template and two specific P₄₅₀C21-oligomers as primers. Oligomer C21-4 (5'-CTG ACT GAT ATC CAT ATG GTC CTC GCA GGG CTG CTG-3') contains 21 nucleotides complementary to C21-sequences from positions 1 to 21 and 18 additional bases to create an EcoRV restriction site and an NdeI restriction site at the ATG initiation codon.

Oligomer C21-5 (5'-AGC TCA GAA TTC CTT CTG GAT GGT CAC-3') is 21 bases complementary to the minus strand upstream the EcoRI-site at position 489.

The PCR was performed as described by Saiki et al (Science 239, 487-491, 1988) with minor modifications. The PCR was performed in a volume of 100 *µ*l containing: 50 mM KCL, 10mM Tris-HCL pH 8.3, 1.5 mM MgCl₂, 0.01 % (w/v) gelatin, 200 µM each dNTP, 1 *µ*M each C21-primer and 10 ng pGBC21-2 template. After denaturation (7' at 100°C) and addition of 2 U Taq-polymerase (Cetus), the reaction mixture was performed to 25 amplification cycles (each: 2' at 55°C, 3' at 72°C, 1' at 94°C) in a DNA-amplifier apparatus (Perkin-Elmer). In the last cycle the denaturation step was omitted. A schematic view of this P₄₅₀C21cDNA amplification is shown in figure 29.

The amplified fragment was digested with EcoRV and EcoRI and inserted by molecular cloning into the appropriate sites of pSP73 (Promega). The obtained plasmid is called pGBC21-3. As shown in figure 30 the 3' P₄₅₀C21-EcoRI fragment of pGBC21-1 was inserted in the right orientation into the EcoRI-site of pGBC21-3. The obtained vector pGBC21-4 was digested with EcoRV and KpnI (KpnI is situated in the multiple cloning site of pSP73) and the fragment containing the full-length P₄₅₀C21cDNA was isolated by gel electrophoresis and inserted into the appropriate sites of pBHA-1 by molecular cloning. The derived plasmid pGBC21-5 is illustrated in figure 31.

### (b) Transformation of Bacillus

The "HpaII" Bacillus promoter was introduced upstream the P₄₅₀C21cDNA gene by digestion pGBC21-5 with the restriction enzyme NdeI, separation of the E.coli part of the shuttle plasmid by agarose gel electrophoresis and subsequent religation and transformation of B.subtilis 1 A40 (BGSC 1 A40) competent cells. Neomycin resistant colonies were analysed to obtain pGBC21-6 (figure 32).

Transformation of the host B.licheniformis T5 (CBS 470.83) was also performed with pGBC21-6. The plasmid remains stable in both Bacillus hosts as revealed by restriction analysis.

### Example 19

### Construction of a P₄₅₀C21cDNA yeast expression vector and transformation to the yeast host Kluyveromyces lactis

### (a) Construction of the expression vector

To derive a suitable expression vector in yeast hosts for bovine P₄₅₀C21, pGBC21-2 was mutated by site directed mutagenesis as described in example 14. For the mutation oligomer C21-6 (5'-CCT CTG CCT GGG TCG ACA AAA ATG GTC CTC GCA GGG-3') was used to create a SalI restriction site and optimal yeast translation signals upstream the ATG initiation codon as indicated in figure 33.

The SalI/EcoR1 DNA fragment of derived plasmid pGBC21-7 was ligated to the 3' P₄₅₀C21-EcoRI-fragment of pGBC21-1 and inserted by molecular cloning into the appropriate sites of pSP73 as indicated in figure 34. Derived pGBC21-8 was cut with SalI and EcoRV (EcoRV site is situated in the multiple cloning site of pSP73) and the DNA fragment containing the full-length P₄₅₀C21cDNA was inserted into the yeast expression vector pGB950. Derived pGBC21-9 is depicted in figure 35.

### (b) Transformation of K.lactis

15 *µ*g of pGBC21-9 was digested with SacII and transformation of K.lactis CBS 2360 was performed as described in example 5(c).

### Example 20

### Molecular cloning of a full-length cDNA encoding the bovine cytochrome P₄₅₀ steroid 11β-hydroxylase (P₄₅₀11β)

A bovine adrenal cortex cDNA library was prepared as described in example 1 with one modification. An additional P₄₅₀11β-specific primer (oligomer 11β-1) with the nucleotide sequence 5'-GGC AGT GTG CTG ACA CGA-3' was added to the reaction mixture of the first strand cDNA synthesis. Oligomer 11β-1 is positioned just downstream the translation stopcodon from position 1530 to 1513. Nucleotide sequences and map positions of mentioned P₄₅₀11β-oligomers are all derived from the P₄₅₀11βcDNA sequence data described by Morohashi et al. (J. Biochem. 102 (3), 559-568, 1987). The cDNA library was screened with a ³²P-labeled oligomer 11β-2 (5'-CCG CAC CCT GGC CTT TGC CCA CAG TGC CAT-3') and is located at the 5' end of the P₄₅₀11βcDNA from position 36 to 1.

Screening with oligomer 11β-2 revealed 6 hybridizing pfu's. These were further purified and analyzed with oligomer 11β-3 (5'-CAG CTC AAA GAG AGT CAT CAG CAA GGG GAA GGC TGT-3', positions 990 to 955). Two out of six showed a positive hybridizing signal with ³²P-labeled oligomer 11β-3.

The EcoRI inserts of both 11β-lambda-gt11 recombinants were subcloned into the EcoRI-site of pTZ18R. One clone with an EcoRI insert of 2.2 kb (PGB11β-1) was further analyzed by restriction enzyme mapping and is shown in figure 36. pGB11β-1 contains all coding P₄₅₀11βcDNA sequences as determined by Morohashi et al.

### Example 21

### Construction of a P₄₅₀C21cDNA Bacillus expression vector and transformation to the bacterial hosts Bacillus subtilis and Bacillus licheniformis

### (a) Construction of the expression vector

A full-length P₄₅₀11βcDNA with modified flanking sequences to the Bacillus expression vector pBHA-1, was obtained by the PCR method (described in example 18) with pGB11β-1 as template and two specific P₄₅₀11β-oligomers as primers.

Oligomer 11β-4 (5'-TTT GAT ATC GAA TTC CAT ATG GGC ACA AGA GGT GCT GCA GCC-3') contains 21 bases complementary to the mature P₄₅₀11βcDNA sequence from position 72 to 93 and 21 bases to create EcoRV, EcoRI and NdeI restriction-sites and ATG initiation codon.

Oligomer 11β-5 (5'-TAA CGA TAT CCT CGA GGG TAC CTA CTG GAT GGC CCG GAA GGT-3) contains 21 bases complementary to the minus P₄₅₀11βcDNA strand upstream the translation stopcodon at position 1511 and 21 bases to create restriction-sites for EcoRV, XhoI and KpnI.

After PCR amplification with above mentioned template and P₄₅₀11β-primers, the amplified fragment (1.45 kb), was digested with EcoRI and KpnI and inserted by molecular cloning into the Bacillus expression vector pBHA-1 cut with EcoRI and KpnI to obtain the vector pGB11β-2 (see figure 36.

### (b) Transformation of Bacillus

The "HpaII" Bacillus promoter was introduced upstream the P₄₅₀11βcDNA sequences by digestion pGB11β-2 with NdeI, separation of the E.coli part of the shuttle plasmid by agarose gel electrophoresis and subsequent religation (as described in example 18) and transformation of B.subtilis 1A40 (BGSC 1A40) competent cells. Neomycin resistant colonies were analysed and the plasmid pGB11β-3 was obtained. The derived plasmid pGB11β-3 was also transmitted to the B.licheniformis host strain T5 (CBS 470.83).

### Example 22

### Construction of a P₄₅₀11βcDNA yeast expression vector and transformation to the yeast host Kluyveromyces lactis

### (a) Construction of the expression cassette

A full-length P₄₅₀11βcDNA with modified flanking sequences to the yeast expression vector pGB950 was obtained by the PCR method (described in example 18) with pGB11β-1 as template and two specific P₄₅₀11β-oligomers as primers.

Oligomer 11β-6 (5'-CTT CAG TCG ACA AAA ATG GGC ACA AGA GGT GCT GCA GCC-3') contains 21 bases complementary to the mature P₄₅₀11βcDNA sequence from position 72 to 93 and 18 additional bases to create a SalI restriction site, an optimal yeast translation signal and an ATG initiation codon.

Oligomer 11β-5 is described in example 21(a). After PCR amplification with above mentioned template and P₄₅₀11β-primers, the amplified fragment (1.45 kb), was digested with SalI and XhoI and inserted by molecular cloning into the yeast expression vector pGB950 cut with SalI to obtain the vector pGB11β-4 (figure 37).

### (b) Transformation of K.lactis

15 *µ*g of pGB11β-4 was cut at the unique SacII site in the lactase promoter and transformation of K.lactis CBS 2360 was performed as described in example 5(c).

### Example 23

Molecular cloning and construction of a full-length cDNA encoding the bovine adrenodoxin (ADX), and subsequent transformation and expression of ADXcDNA in the yeast Kluyveromyces lactis

### (a) Molecular cloning of ADX

A full-length ADXcDNA, with 5' and 3' flanking sequences modified to the yeast expression vector pGB950, was directly obtained from a bovine adrenal cortex mRNA/cDNA pool (for detailed description see example 1) by amplification using the PCR method (see example 18).

For the ADXcDNA amplification two synthetic oligomer primers were synthesized.

Oligomer ADX-1 (5'-CTT CAG TCG ACA AAA ATG AGC AGC TCA GAA GAT AAA ATA-3') containing 21 bases complementary to the 5' end of the mature ADXcDNA sequence as described by Okamura et al (Proc. Natl. Acad. Sci. USA, 82, 5705-5709, 1985) from positions 173 to 194. The oligomer ADX-1 contains at the 5' end 18 additional nucleotides to create a SalI restriction site, an optimal yeast translation signal and an ATG initiation codon.

The oligomer ADX-2 (5'-TGT AAG GTA CCC GGG ATC CTT ATT CTA TCT TTG AGG AGT T-3') is complementary to the 3'end of the minus strand of ADXcDNA from position 561 to 540 and contains additional nucleotides for creating restriction sites for BamHI, SmaI and KpnI.

The PCR was performed as described in example 18 with 1*µ* M of each ADX-primers and 10 *µ*l mRNA/cDNA mixture (as described in example 1) as template.

A schematic view of this ADXcDNA amplification is shown in figure 38.

The amplified fragment contains a full-length ADXcDNA sequence with modified flankings, which was characterized by restriction-site analysis and nucleotide sequencing.

### (b) Construction of the expression vector

The amplified ADXcDNA fragment was digested with SalI and SmaI and inserted by molecular cloning into the yeast expression vector pGB950 cut with SalI and EcoRV. The derived plasmid pGBADX-1 is depicted in figure 38.

### (c) Transformation of K.lactis

15 *µ*g of pGBADX-1 was cut at the unique SacII-site in the lactase promoter and transformation of K.lactis CBS 2360 was performed as described in example 5(c).

### (d) Analysis of the transformants

Two transformants, ADX-101 and ADX-102 and the control strain CBS 2360 were selected for further analysis. The strains were grown in YEPD-medium for about 64 hrs at 30°C. Total cellular protein was isolated as described in example 5(d). From the supernatants 8 *µ*l samples were taken for analysis on immunoblots (see figure 39, lane 3, 4 and 5).

The results show that a protein of the expected length (14 kDa) is expressed in K.lactis cells transformed with pGBADX-1.

The in vitro ADX-activity of transformant ADX-102 is described in example 24.

### Example 24

### In vitro activity of adrenodoxin obtained from Kluyveromyces lactis ADX-102

K.lactis ADX-102, obtained as described in example 23, and control strain K.lactis CBS 2360 were grown in 100 ml YEPD medium (1% yeast extract, 2% peptone, 2% glucose monohydrate) containing 2.5 ml of a 6.7% (w/w) yeast nitrogen base (Difco laboratories) solution and 100 mg l⁻¹ of geneticin (G418 sulphate; Gibco Ltd.), for 56 hours at 30°C. The cells were collected by centrifugation (4000xg, 15 minutes), resuspended in a physiological salt solution and washed with a phosphate buffer (pH 7.0, 50 mM). After centrifugation (4000xg, 15 minutes) the pellet was resuspended in a phosphate buffer (pH 7.0, 50 mM) resulting in a suspension containing 0.5 g cell wet weight/ml. The cells were disrupted using a Braun MSK Homogenizer (6 x 15 seconds, 0.45 - 0.50 mm glass beads). Unbroken cells were removed by centrifugation (4000xg, 15 minutes). The cell-free extracts (40 mg protein/ml) were stored at -20°C.

ADX activity, i.e. electrontransfer capacity from adrenodoxin reductase to cytochrome P₄₅₀SCC, in the cell-free extracts was determined by a P₄₅₀SCC activity assay. The assay mixture consisted of the following solutions:
Solution A (natural P₄₅₀SCC electron donating system with the exception of ADX): a 50 mM potassium phosphate buffer (pH 7.0), containing 3 mM of EDTA, 2 *µ*M of adrenodoxin reductase (purified from bovine adrenal cortex), 1 mM NADPH (electron donor), 15 mM glucose-6-phosphate and 16 units/ml glucose-6-phosphate-dehydrogenase (NADPH regenerating system).
Solution B (substrate and enzyme): a micellar solution of 75 *µ*M of cholesterol (doubly radiolabeled with [26,27-¹⁴C] cholesterol (40 Ci/mol) and [7α-³H] cholesterol (400 Ci/mol)) and 1.5 *µ*M of P₄₅₀SCC (purified from bovine adrenal cortex) in 10% (v/v) Tergitol™ NP 40/ethanol (1:1, v/v).

The assay was started by mixing 75 *µ*l of solution A with 50 *µ*l of solution B and 125 *µ*l of cell-free extract or 125 *µ*l of a potassium phosphate buffer (50 mM, pH 7.0) containing 10 *µ*M ADX (purified from bovine adrenal cortex). The mixture was stirred gently at 30°C. Samples were drawn after 15 minutes of incubation and diluted with 100 *µ*l of water. From a sample substrate and product(s) were extracted with 100 *µ*l of methanol and 150 *µ*l of chloroform.
After centrifugation (5000xg, 2 minutes) the chloroform layer was collected and dried. The dry residue was dissolved in 50 *µ*l of acetone, containing 0.5 mg of a steroid mixture (cholesterol, pregnenolone and progesterone (1:1:1, w/w/w)) and subsequently 110 *µ*l of concentrated formic acid was added. The suspension was heated for 15 minutes at 120°C. Hereafter the ¹⁴C/³H ratio was determined by double label liquid scintillation counting. The ratio is a direct measure for the side chain cleavage reaction, because the ¹⁴C-labeled side chain is evaporated from the mixture as isocaprylic acid during the heating procedure.

Using this assay ADX electron carrier activity could easily be demonstrated in the cell-free extract of K.lactis ADX102. In the assays with cell-free extract of K.lactis ADX-102 or with purified ADX, the side chain of the cholesterol was cleaved within 15 minutes in a yield of 50%, whereas in the assay with cell-free extract of the control strain K.lactis CBS 2360 no side chain cleavage could be detected.

### Example 25

### Molecular cloning and construction of a full-length cDNA encoding the bovine adrenodoxin oxidoreductase (ADR), and subsequent transformation of ADRcDNA in the yeast Kluyveromyces lactis

### (a) Molecular cloning of adrenodoxin oxidoreductase

A bovine adrenal cortex cDNA library was prepared as described in example 1 with one modification. An additional ADR-specific primer (oligomer ADR-1) with the nucleotide sequence 5'-GGC TGG GAT CTA GGC-3' was added to the reaction mixture of the first strand cDNA synthesis. Oligomer ADR-1 is located just downstream the translation stopcodon from position 1494 to 1480. Nucleotide sequences and map positions of mentioned ADR-oligomers are all derived from the ADRcDNA sequence data described by Nonaka et al, Biochem. Biophys. Res. Comm. 145(3), 1239-1247, 1987.

Obtained cDNA library was screened with a ³²P-labeled oligomer ADR-2 (5'-CAC CAC ACA GAT CTG GGG GGT CTG CTC CTG TGG GGA-3').

4 hybridizing pfu's were identified and subsequently purified. However only 1 pfu showed also a positive signal with oligomer ADR-3 (5'-TTC CAT CAG CCG CTT CCT CGG GCG AGC GGC CTC CCT-3'), which is located in the middle of the ADRcCDNA (position 840 to 805). The ADRcDNA insert (approx. 2 kb) was molecular cloned into the EcoRI-site of pTZ18R.

The obtained plasmid pGBADR-1 contains a full-length ADRcDNA as revealed by restriction enzyme mapping and nucleotide sequencing. The physical map of pGBADR-1 is illustrated in figure 40.

### (b) Construction of the expression cassette

A full-length ADRcDNA with modified flanking sequences to the yeast expression vector pGB950 was obtained by the PCR method (see example 18) with pGBADR-1 as template and two specific ADR-oligomers as primers.

Oligomer ADR-4 (5'-CGA GTG TCG ACA AAA ATG TCC ACA CAG GAG CAG ACC-3') contains 18 bases complementary to the mature ADRcDNA sequences from position 96 to 114 and 18 bases to introduce a SalI restriction site, an optimal yeast translation signal, and an ATG initiation codon.

Oligomer ADR-5 (5'-CGT GCT CGA GGT ACC TCA GTG CCC CAG CAG CCG CAG-3') contains 18 bases complementary to the minus strand of ADRcDNA upstream the translation stopcodon at position 1479 and 15 bases to create KpnI and XhoI restriction sites for molecular cloning in various expression vectors.

After amplification with above mentioned template and ADR primers, the amplified fragment (1.4 kb) was digested with SalI and XhoI and inserted by molecular cloning into the yeast expression vector pGB950 cut with SalI and XhoI.

The derived plasmid pGBADR-2 is illustrated in figure 40.

### (c) Transformation of K.lactis

15 *µ*g of pGBADR-2 was cut at the unique SacII-site in the lactase promoter and transformation of K.lactis CBS 2360 was performed as described in example 5(c).

### Example 26

### Molecular cloning of a full-length cDNA encoding bovine NADPH-cytochrome P₄₅₀ reductase (RED)

The bovine adrenal cortex cDNA library described in example 1 was screened with a ³²P-labeled synthetic oligomer 5'-TGC CAG TTC GTA GAG CAC ATT GGT GCG TGG CGG GTT AGT GAT GTC CAG GT-3', specific for a conserved amino acid region within rat-, porcine- and rabbit RED as described by Katagari et al. (J. Biochem., 100, 945-954, 1986) and Murakami et al. (DNA, 5, 1-10, 1986).

Five hybridizing pfu's were obtained and further characterized by restriction enzyme mapping and nucleotide sequencing. A full-length REDcDNA was inserted into expression vectors and transformed to appropriate hosts as mentioned in examples 2, 3 and 6.

### Example 27

### Construction, transformation and expression of an expression cassette encoding the proteins P₄₅₀SCC and ADX in the yeast Kluyveromyces lactis

### (a) Construction of the expression cassette

The expression cassette pGBADX-1 (see example 23) was digested with SacII and HindIII (partially) and sticky ends were filled in using Klenow DNA polymerase. The DNA fragment comprising a part of the lactase promoter (but still functional), the coding ADX sequence and the lactase terminator was separated and isolated by agarose-gel electrophoresis and subsequently inserted into pGBSCC-7, which was first linearized by XbaI digestion (see example 5(b)) and sticky ends filled in using Klenow DNA polymerase. The construction was set up in such a manner that a unique restriction site (SacII) is obtained, which is necessary to transfer the plasmid to K.lactis.
This unique SacII restriction site is located in the lactase promoter sequence flanking the SCC sequence, as the SacII restriction site in the lactase promoter flanking the ADX sequence is destroyed by the fill-in reaction. The obtained expression cassette pGBSCC/ADX-1 contains the coding sequence for SCC as well as for ADX, each driven by the lactase promoter.

### (b) Transformation of K.lactis

Transformation of K.lactis CBS 2360 was performed as described in example 5(c) with 15 *µ*g pGBSCC/ADX-1, linearized at the unique SacII restriction site. One transformant (SCC/ADX-101) was selected for SCC and ADX expression studies.

### (c) Analysis of the transformant K.lactis SCC/ADX-101

Cellular protein fractions were prepared from cultures of the SCC/ADX-101 and the control strain CBS 2360 as described in example 5(d) and analyzed by SDS/PAGE and Western-blotting. The blot was probed with antibodies specific for SCC and ADX, respectively.
Compared to the control strain, the cellular protein fraction of transformant SCC/ADX-101 shows two additional bands of expected length (53 and 14 kDa, respectively) showing the expression of both proteins SCC and ADX. Expression levels of both proteins in transformant SCC/ADX-101 are comparable with levels observed in transformants expressing only one protein (for SCC see figure 15A, lane 3, and for ADX figure 39, lane 5).

The in vitro SCC and ADX activity of transformant SCC/ADX-101 is described in example 28.

### Example 28

### In vitro activity of P₄₅₀SCC and adrenodoxin obtained from Kluyveromyces lactis SCC/ADX-101

K.lactis SCC/ADX-101 obtained as described in example 27 and control strain K.lactis SCC-101 as described in example 5(d) were grown in 1 l of YEPD medium (1% yeast extract, 2% peptone, 2% glucose monohydrate) containing 100 mg l⁻¹ of geneticin (G418 sulphate; Gibco Ltd.), for 72 hours at 30°C. The cells were collected by centrifugation (4000xg, 15 minutes), resuspended in a physiological salt solution and washed with a phosphate buffer (pH 7.5, 75 mM). After centrifugation (4000xg, 15 minutes) the pellet was resuspended in a phosphate buffer (pH 7.5, 75 mM) resulting in a suspension containing 0.5 g cell wet weight/ml. The cells were disrupted using a Braun MSK Homogenizer (6 x 15 seconds, 0.45 - 0.50 mm glass beads). Unbroken cells were removed by centrifugation (4000xg, 15 minutes).

In the cell-free extracts the activity of the protein complex P₄₅₀SCC/ADX was assayed, by determining the cholesterol side-chain cleaving reaction in the presence of NADPH and ADR. The assay mixture consisted of the following solutions:
Solution A (natural P₄₅₀SCC electron donating system with the exception of ADX): a 50 mM potassium phosphate buffer (pH 7.0), containing 3 mM of EDTA, 2 *µ*M of adrenodoxin reductase (purified from bovine adrenal cortex), 1 mM NADPH (electron donor), 15 mM glucose-6-phosphate and 16 units/ml glucose-6-phosphate-dehydrogenase (NADPH regenerating system).
Solution B (substrate): a micellar solution of 37,5 *µ*M of cholesterol (doubly radiolabeled with [26,27-¹⁴C] cholesterol (40 Ci/mol) and [7α-³H] cholesterol (400 Ci/mol)) in 10% (v/v) Tergitol™ NP 40/ethanol (1:1, v/v).

The assay was started by mixing 75 *µ*l of solution A with 50 *µ*l of solution B and 125 *µ*l of cell-free extract. The mixture was stirred gently at 30°C. Samples were drawn after 60 minutes of incubation and diluted with 100 *µ*l of water. From a sample substrate and product(s) were extracted with 100 *µ*l of methanol and 150 *µ*l of chloroform.
After centrifugation (5000xg, 2 minutes) the chloroform layer was collected and dried). The dry residu was dissolved in 50 *µ*l of acetone, containing 0.5 mg of a steroid mixture (cholesterol, pregnenolone and progesterone (1:1:1, w/w/w)) and subsequently 110 *µ*l of concentrated formic acid was added.
The suspension was heated for 15 minutes at 120°C. Hereafter the ¹⁴C/³H ratio was determined by double label liquid scintillation counting. The ratio is a direct measure for the side-chain cleaving reaction, because the ¹⁴C-labeled side-chain is evaporated from the mixture as isocaprylic acid during the heating procedure.

Using this assay cholesterol side-chain cleaving activity was demonstrated in the cell-free extract of K.lactis SCC/ADX-101, whereas in the cell-free extract of K.lactis SCC-101 no activity was detectable.
By means of HPLC-analysis, the reaction product produced by a cell-free extract of K.lactis SCC/ADX-101 was identified as pregnenolone.

## Claims

1. Use of an expression cassette in multigenic systems for conducting multi-step conversions as depicted in figure 1, wherein said expression cassette is operable in a non mammalian recombinant host, characterized in that the expression cassette comprises a heterologous DNA coding sequence encoding a protein, which is functional, alone or in cooperation with one or more additional proteins, of catalyzing an oxidation step in the biological pathway for conversion of cholesterol into hydrocortisone, which step is selected from the group consisting of :
the conversion of cholesterol to pregnenolone ;
the conversion of pregnenolone to progesterone ;
the conversion of progesterone to 17α-hydroxy-progesterone ;
the conversion of 17α-hydroxyprogesterone to cortexolone and
the conversion of cortexolone to hydrocortisone,
and the corresponding control sequences effective in said host.

2. Use of an expression cassette according to claim 1, characterized in that the protein is one selected from the group consisting of :
side-chain cleaving enzyme (P₄₅₀SCC) ;
adrenodoxin (ADX) ;
adrenodoxin reductase (ADR) ;
3β-hydroxysteroid dehydrogenase/isomerase (3β-HSD) ;
steroid-17α-hydroxylase (P₄₅₀17α) ;
NADPH cytochrome P₄₅₀ reductase (RED) ;
steroid-21-hydroxylase (P₄₅₀C21) and
steroid-11β-hydroxylase (P₄₅₀11β).

3. Use of an expression cassette accordinq to claim 2, characterized in that the heterologous DNA coding sequence originate from bovine species.

4. Use of an expression cassette pGBSCC-5 according to claim 3, characterized in that the heterologous DNA encodes the enzyme P₄₅₀SCC and that the expression cassette depicted in figure 8 is operative in species of Bacillus.

5. Use of an expression cassette pGBSCC-17 according to claim 3, characterized in that the heterologous DNA encodes the enzyme P₄₅₀SCC and that the expression cassette depicted in figure 10 is operative in E. coli.

6. Use of an expression cassette according to claim 3,
characterized in that the heterologous DNA encodes the enzyme P₄₅₀SCC and that the expression cassette operative in Kluyveromyces lactis is taken from the group consisting of pGBSCC-7 depicted in figure 14, pGBSCC-12 depicted in figure 18 and pGBSCC-15 depicted in figure 20 or that the expression cassette operative in Saccharomyces cerevisiae is taken form the group consisting of pGBSCC-10 depicted in figure 17, pGBSCC-13 depicted in figure 19 and pGBSCC-16 depicted in figure 21.

7. Use of an expression cassette pGB17α-5 according to claim 3, characterized in that the heterologous DNA encodes the enzyme P₄₅₀17α and that the expression cassette depicted in figure 24 is operative in Kluyveromyces lactis.

8. Use of an expression cassette pGB17α-8 according to claim 3, characterized in that the heterologous DNA encodes the enzyme P₄₅₀17α and that the expression cassette depicted in figure 27 is operative in species of Bacillus.

9. Use of an expression cassette pGB11β-4 according to claim 3, characterized in that the heterologous DNA encodes the enzyme P₄₅₀11β and that the expression cassette depicted in figure 37 is operative in Kluyveromyces lactis.

10. An expression cassette, operable in a non mammalian recombinant host, comprising a heterologous DNA coding sequence encoding a protein, which is functional, alone or in cooperation with one or more additional proteins, of catalyzing an oxidation step in the biological pathway for conversion of cholesterol into hydrocortisone, which step is selected from the group consisting of :
the conversion of cholesterol to pregnenolone ;
the conversion of pregnenolone to progesterone ;
the conversion of progesterone to 17α-hydroxy-progesterone ;
the conversion of 17α-hydroxyprogesterone to cortexolone and
the conversion of cortexolone to hydrocortisone,
and the corresponding control sequences effective in said host, characterized in that the protein is one selected from the group consisting of
side-chain cleaving enzyme (P₄₅₀SCC) ;
adrenodoxin (ADX) ; adrenodoxin reductase (ADR) ;
3β-hydroxysteroid dehydrogenase/isomerase (3β-HSD) ;
steroid-17α-hydroxylase (P₄₅₀17α) ;
NADPH cytochrome P₄₅₀ reductase (RED) ;
steroid-21-hydroxylase (P₄₅₀C21) and
steroid-11β-hydroxylase (P₄₅₀11β),
and that the heterologous DNA encodes at least one additional protein of the said group of proteins.

11. An expression cassette according to claim 10, operable in a non mammalian recombinant host, comprising a heterologous DNA coding sequence encoding a protein, which is functional, alone or in cooperation with one or more additional proteins, of catalyzing an oxidation step in the biological pathway for conversion of cholesterol into hydrocortisone, which step is selected from the group consisting of :
the conversion of cholesterol to pregnenolone ;
the conversion of pregnenolone to progesterone ;
the conversion of progesterone to 17α-hydroxy-progesterone ;
the conversion of 17α-hydroxyprogesterone to cortexolone and
the conversion of cortexolone to hydrocortisone,
and the corresponding control sequences effective in said host, characterized in that the protein is one selected from the group consisting of
side-chain cleaving enzyme (P₄₅₀SCC) ;
adrenodoxin (ADX) ; adrenodoxin reductase (ADR) ;
3β-hydroxysteroid dehydrogenase/isomerase (3β-HSD) ;
steroid-17α-hydroxylase (P₄₅₀17α) ;
NADPH cytochrome P₄₅₀ reductase (RED) ;
steroid-21-hydroxylase (P₄₅₀C21 ) and
steroid-11β-hydroxylase (P₄₅₀11β),
and wherein the heterologous DNA encoding said additional protein is one additional heterologous DNA with its own effective control effective sequences encoding a protein from the said group of proteins.

12. An expression cassette according to claims 10 or 11, characterized in that the heterologous DNA encodes bovine P₄₅₀SCC and bovine ADX.

13. An expression cassette operable in a non mammalian recombinant host, comprising a heterologous DNA coding sequence encoding at least two proteins, which are functional, alone or in cooperation with one or more additional proteins, of catalyzing at least two oxidation steps selected from the group consisting of :
the conversion of cholesterol to pregnenolone ;
the conversion of pregnenolone to progesterone ;
the conversion of progesterone to 17α-hydroxy-progesterone ;
the conversion of 17α-hydroxyprogesterone to cortexolone and
the conversion of cortexolone to hydrocortisone,
and the corresponding control sequences effective in said host.

14. An expression cassette according to claim 13, operable in a non mammalian recombinant host, comprising a heterologous DNA coding sequence encoding at least two proteins, which are functional, alone or in cooperation with one or more additional proteins, of catalyzing at least two oxidation steps selected from the group consisting of :
the conversion of cholesterol to pregnenolone ;
the conversion of pregnenolone to progesterone ;
the conversion of progesterone to 17α-hydroxy-progesterone ;
the conversion of 17α-hydroxyprogesterone to cortexolone and
the conversion of cortexolone to hydrocortisone,
characterized in that the heterologous DNA encoding the second protein has its own effective control sequences.

15. An expression cassette according to claim 13 or 14, characterized in that the protein is one selected from the group consisting of :
side-chain cleaving enzyme (P₄₅₀SCC) ;
adrenodoxin (ADX) ; adrenodoxin reductase (ADR) ;
3β-hydroxysteroid dehydrogenase/isomerase (3β-HSD) ;
steroid-17α-hydroxylase (P₄₅₀17α) ;
NADPH cytochrome P₄₅₀ reductase (RED) ;
steroid-21-hydroxylase (P₄₅₀C21) and
steroid-11β-hydroxylase (P₄₅₀11β).

16. An expression cassette accordinq to claim 15, characterized in that the heterologous DNA coding sequences originate from bovine species.

17. A recombinant host cell and progeny thereof comprising cells of micro-organisms, plants or non mammalian animals and containing an expression cassette with heterologous DNA characterized in that the expression cassette is one defined in any one of claims 10-16.

18. A recombinant host cell and progeny thereof according to claim 17, characterized in that the host is a micro-organism.

19. A recombinant host cell and progeny thereof according to claim 18, characterized in that the host is a species of Saccharomyces, Kluyveromyses or Bacillus or is Escherichia coli.

20. A recombinant host cell and progeny thereof according to any one of claims 17-19 and containing at least two expression cassettes as defined in any one of claims 10-16.

21. A process for the preparation of a mixture of exogenous proteins by a recombinant cell in a nutrient medium under conditions enabling the enzymes to be formed and accumulated in the culture, characterized in that the recombinant cell is a recombinant host cell as defined in claim 20.

22. A process for selective biochemical oxidation in vitro, which process comprises :
incubating the compound to be oxidized in the presence of proteins under conditions which permit said oxidation and the accumulation of the oxidized compound in the culture liquid, followed by recovering the oxidized compound, characterized in that the proteins have been produced by the process of claim 21.

23. A process for oxidizing a selected compound, which process comprises :
culturing recombinant cells in the presence of said compound under conditions wherein the desired oxidation occurs and the oxidized compound accumulates in the culture liquid, followed by recovering the oxidized compound, characterized in that the recombinant cells are the recombinant host cells of any one of claims 17-20.

24. A process according to claims 22 or 23,
characterized in that the oxidation is one selected from the group consisting of :
cleaving the side-chain of a sterol compound to pregnenolone ;
the conversion of pregnenolone to progesterone ;
the conversion of progesterone to 17α-hydroxyprogesterone ;
the conversion of 17α-hydroxyprogesterone to cortexolone and
the conversion of cortexolone to hydrocortisone.

25. A process according to claim 24, characterized in that the oxidation is cleaving the side-chain of a sterol compound resulting in pregnenolone.

26. A process according to claim 24, characterized in that the oxidation is the 17α-hydroxylation of progesterone.

27. A process according to claim 24, characterized in that at least two oxidations from said group are carried out on the same substrate molecule in one step.

## Patentansprüche

1. Verwendung einer Expressionskassette in Multigensystemen zur Durchführung von mehrstufigen Umwandlungen, wie in Figur 1 dargestellt, wobei die Expressionskassette in einem rekombinanten Nicht-Säuger-Wirt funktionsfähig ist, dadurch **gekennzeichnet**, dass die Expressionskassette eine heterologe DNA-Codierungssequenz, die ein Protein codiert, das allein oder in Kooperation mit einem oder mehreren weiteren Protein(en) funktionsfähig ist, eine Oxidationsstufe in dem biologischen Stoffwechselweg zur Umwandlung von Cholesterin in Hydrocortison zu katalysieren, wobei die Stufe aus der Gruppe
Umwandlung von Cholesterin in Pregnenolon;
Umwandlung von Pregnenolon in Progesteron;
Umwandlung von Progesteron in 17α-Hydroxyprogesteron;
Umwandlung von 17α-Hydroxyprogesteron in Cortexolon; und Umwandlung von Cortexolon in Hydrocortison
ausgewählt ist, und die entsprechenden in dem Wirt wirksamen Kontrollsequenzen, umfasst.

2. Verwendung einer Expressionskassette nach Anspruch 1, dadurch **gekennzeichnet**, dass das Protein eines ausgewählt aus der Gruppe
seitenkettenspaltendes Enzym (P₄₅₀SCC) ;
Adrenodoxin (ADX);
Adrenodoxinreduktase (ADR) ;
3β-Hydroxysteroiddehydrogenase/Isomerase (3β-HSD) ;
Steroid-17α-hydroxylase (P₄₅₀17α) ;
NADPH-Cytochrom-P₄₅₀-reduktase (RED) ;
Steroid-21-hydroxylase (P₄₅₀C21) ; und
Steroid-11β-hydroxylase (P₄₅₀11β)
ist.

3. Verwendung einer Expressionskassette nach Anspruch 2, dadurch **gekennzeichnet**, dass die heterologe DNA-Codierungssequenz aus einer Rinderart stammt.

4. Verwendung einer Expressionskassette pGBSCC-5 nach Anspruch 3, dadurch **gekennzeichnet**, dass die heterologe DNA das Enzym P₄₅₀SCC codiert und dass die in Figur 8 dargestellte Expressionskassette in der Art Bacillus funktionsfähig ist.

5. Verwendung einer Expressionskassette pGBSCC-17 nach Anspruch 3, dadurch **gekennzeichnet**, dass die heterologe DNA das Enzym P₄₅₀SCC codiert und dass die in Figur 10 dargestellte Expressionskassette in E. coli funktionsfähig ist.

6. Verwendung einer Expressionskassette nach Anspruch 3, dadurch **gekennzeichnet**, dass die heterologe DNA das Enzym P₄₅₀SCC codiert und dass die in Kluyveromyces lactis funktionsfähige Expressionskassette aus der Gruppe bestehend aus pGBSCC-7, dargestellt in Figur 14, pGBSCC-12, dargestellt in Figur 18, und pGBSCC-15, dargestellt in Figur 20, stammt oder dass die in Saccharomyces cerevisisiae funktionsfähige Expressionskassette aus der Gruppe bestehend aus pGBSCC-10, dargestellt in Figur 17, pGBSCC-13, dargestellt in Figur 19, und pGBSCC-16, dargestellt in Figur 21, stammt.

7. Verwendung einer Expressionskassette pGB17α-5 nach Anspruch 3, dadurch **gekennzeichnet**, dass die heterologe DNA das Enzym P₄₅₀17α codiert und dass die in Figur 24 dargestellte Expressionskassette in Kluyveromyces lactis funktionsfähig ist.

8. Verwendung einer Expressionskassette pGB17α-8 nach Anspruch 3, dadurch **gekennzeichnet**, dass die heterologe DNA das Enzym P₄₅₀17α codiert und dass die in Figur 27 dargestellte Expressionskassette in der Art Bacillus funktionsfähig ist.

9. Verwendung einer Expressionskassette pGB11β-4 nach Anspruch 3, dadurch **gekennzeichnet**, dass die heterologe DNA das Enzym P₄₅₀11β codiert und dass die in Figur 37 dargestellte Expressionskassette in Kluyveromyces lactis funktionsfähig ist.

10. Expressionskassette, die in einem rekombinanten Nicht-Säuger-Wirt funktionsfähig ist, umfassend eine heterologe DNA-Codierungssequenz, die ein Protein codiert, das allein oder in Kooperation mit einem oder mehreren weiteren Protein(en) funktionsfähig ist, eine Oxidationsstufe in dem biologischen Stoffwechselweg zur Umwandlung von Cholesterin in Hydrocortison zu katalysieren, wobei die Stufe aus der Gruppe bestehend aus
Umwandlung von Cholesterin in Pregnenolon;
Umwandlung von Pregnenolon in Progesteron;
Umwandlung von Progesteron in 17α-Hydroxyprogesteron;
Umwandlung von 17α-Hydroxyprogesteron in Cortexolon; und
Umwandlung von Cortexolon in Hydrocortison
ausgewählt ist und die entsprechende Kontrollsequenz, die in dem Wirt wirksam ist, dadurch **gekennzeichnet,** dass das Protein eines ausgewählt aus der Gruppe bestehend aus
seitenkettenspaltendem Enzym (P₄₅₀SCC) ;
Adrenodoxin (ADX);
Adrenodoxinreduktase (ADR);
3β-Hydroxysteroiddehydrogenase/Isomerase (3β-HSD) ;
Steroid-17α-hydroxylase (P₄₅₀17α) ;
NADPH-Cytochrom-P₄₅₀-reduktase (RED) ;
Steroid-21-hydroxylase (P₄₅₀C21); und
Steroid-11β-hydroxylase (P₄₅₀11β)
ist und dass die heterologe DNA mindestens ein weiteres Protein der Gruppe von Proteinen codiert.

11. Expressionskassette nach Anspruch 10, die in einem rekombinanten Nicht-Säuger-Wirt funktionsfähig ist, umfassend eine heterologe DNA-Codierungssequenz, die ein Protein codiert, das allein oder in Kooperation mit einem oder mehreren weiteren Protein(en) funktionsfähig ist, eine Oxidationsstufe in dem biologischen Stoffwechselweg zur Umwandlung von Cholesterin in Hydrocortison zu katalysieren, wobei die Stufe ausgewählt ist aus der Gruppe bestehend aus
Umwandlung von Cholesterin in Pregnenolon;
Umwandlung von Pregnenolon in Progesteron;
Umwandlung von Progesteron in 17α-Hydroxyprogesteron;
Umwandlung von 17α-Hydroxyprogesteron in Cortexolon; und
Umwandlung von Cortexolon in Hydrocortison,
und die entsprechenden Kontrollsequenzen, die in dem Wirt wirksam sind, dadurch **gekennzeichnet**, dass das Protein eines ausgewählt aus der Gruppe bestehend aus
seitenkettenspaltendem Enzym (P₄₅₀SCC) ;
Adrenodoxin (ADX);
Adrenodoxinreduktase (ADR);
3β-Hydroxysteroiddehydrogenase/Isomerase (3β-HSD) ;
Steroid-17α-hydroxylase (P₄₅₀17α) ;
NADPH-Cytochrom-P₄₅₀-reduktase (RED);
Steroid-21-hydroxylase (P₄₅₀C21) ; und
Steroid-11β-hydroxylase (P₄₅₀11β)
ist und dass die heterologe DNA, die das zusätzliche Protein codiert, eine zusätzliche heterologe DNA mit ihren eigenen wirksamen Kontrollsequenzen ist, die ein Protein aus der Gruppe von Proteinen codiert.

12. Expressionskassette nach Anspruch 10 oder 11, dadurch **gekennzeichnet**, dass die heterologe DNA Rinder-P₄₅₀SCC und Rinder-ADX codiert.

13. Expressionskassette, die in einem rekombinanten Nicht-Säuger-Wirt funktionsfähig ist, umfassend eine heterologe DNA-Codierungssequenz, die mindestens zwei Proteine codiert, die allein oder in Kooperation mit einem oder mehreren weiteren Proteinen funktionsfähig sind, mindestens zwei Oxidationsstufen, ausgewählt aus der Gruppe bestehend aus
Umwandlung von Cholesterin in Pregnenolon;
Umwandlung von Pregnenolon in Progesteron;
Umwandlung von Progesteron in 17α-Hydroxyprogesteron;
Umwandlung von 17α-Hydroxyprogesteron in Cortexolon; und
Umwandlung von Cortexolon in Hydrocortison,
zu katalysieren und die entsprechenden Kontrollsequenzen, die in dem Wirt wirksam sind.

14. Expressionskassette nach Anspruch 13, die in einem rekombinanten Nicht-Säuger-Wirt funktionsfähig ist, umfassend eine heterologe DNA-Codierungssequenz, die mindestens zwei Proteine codiert, die allein oder in Kooperation mit einem oder mehreren weiteren Protein(en) funktionsfähig sind, mindestens zwei Oxidationsstufen, ausgewählt aus der Gruppe bestehend aus
Umwandlung von Cholesterin in Pregnenolon;
Umwandlung von Pregnenolon in Progesteron;
Umwandlung von Progesteron in 17α-Hydroxyprogesteron;
Umwandlung von 17α-Hydroxyprogesteron in Cortexolon; und
Umwandlung von Cortexolon in Hydrocortison
zu katalysieren, dadurch **gekennzeichnet**, dass die heterologe DNA, die das zweite Protein codiert, ihre eigenen wirksamen Kontrollsequenzen besitzt.

15. Expressionskassette nach Anspruch 13 oder 14, dadurch **gekennzeichnet**, dass das Protein eines ausgewählt aus der Gruppe bestehend aus
seitenkettenspaltendem Enzym (P₄₅₀SCC) ;
Adrenodoxin (ADX);
Adrenodoxinreduktase (ADR);
3β-Hydroxysteroiddehydrogenase/Isomerase (3β-HSD) ;
Steroid-17α-hydroxylase (P₄₅₀17α) ;
NADPH-Cytochrom-P₄₅₀-reduktase (RED);
Steroid-21-hydroxylase (P₄₅₀C21); und
Steroid-11β-hydroxylase (P₄₅₀11β)
ist.

16. Expressionskassette nach Anspruch 15, dadurch **gekennzeichnet,** dass die heterologe DNA-Codierungssequenz aus einer Rinderart stammt.

17. Rekombinante Wirtszelle und Nachkommen davon, umfassend Zellen von Mikroorganismen, Pflanzen und Nicht-Säugern, enthaltend eine Expressionskassette mit heterologer DNA, dadurch **gekennzeichnet**, dass die Expressionskassette eine ist, die in den Ansprüchen 10 bis 16 definiert ist.

18. Rekombinante Wirtszelle und Nachkommen davon nach Anspruch 17, dadurch **gekennzeichnet**, dass der Wirt ein Mikroorganismus ist.

19. Rekombinante Wirtszelle und Nachkommen davon nach Anspruch 18, dadurch **gekennzeichnet**, dass der Wirt eine Art von Saccharomyces, Kluyveromyces oder Bacillus oder Escherichia coli ist.

20. Rekombinante Wirtszelle und Nachkommen davon nach einem der Ansprüche 17 bis 19 und enthaltend mindestens zwei Expressionskassetten wie in einem der Ansprüche 10 bis 16 definiert.

21. Verfahren zur Herstellung eines Gemisches aus exogenen Proteinen durch eine rekombinante Zelle in einem Nährmedium unter Bedingungen, die die Bildung und Anreicherung von Enzymen in der Kultur erlauben, dadurch **gekennzeichnet,** dass die rekombinante Zelle eine rekombinante Wirtszelle wie in Anspruch 20 definiert ist.

22. Verfahren zur selektiven biochemischen Oxidation in vitro, wobei man die zu oxidierende Verbindung in Gegenwart von Proteinen unter Bedingungen inkubiert, die die Oxidation und die Anreicherung der oxidierten Verbindung in der Kulturflüssigkeit erlauben, und dann die oxidierte Verbindung isoliert, dadurch **gekennzeichnet,** dass die Proteine nach dem Verfahren von Anspruch 21 produziert wurden.

23. Verfahren zur Oxidation einer ausgewählten Verbindung, wobei man rekombinante Zellen in Gegenwart der Verbindung unter Bedingungen züchtet, bei denen die gewünschte Oxidation auftritt und die oxidierte Verbindung sich in der Kulturflüssigkeit anreichert, und dann die oxidierte Verbindung isoliert, dadurch **gekennzeichnet,** dass die rekombinanten Zellen die rekombinanten Wirtszellen nach einem der Ansprüche 17 bis 20 sind.

24. Verfahren nach den Ansprüchen 22 oder 23, dadurch **gekennzeichnet**, dass die Oxidation eine ausgewählt aus der Gruppe bestehend aus
Spaltung der Seitenkette einer Sterinverbindung in Pregnenolon;
Umwandlung von Pregnenolon in Progesteron;
Umwandlung von Progesteron in 17α-Hydroxyprogesteron;
Umwandlung von 17α-Hydroxyprogesteron in Cortexolon; und
Umwandlung von Cortexolon in Hydrocortison
ist.

25. Verfahren nach Anspruch 24, dadurch **gekennzeichnet,** dass die Oxidation die Spaltung der Seitenkette einer Sterinverbindung zu Pregnenolon ist.

26. Verfahren nach Anspruch 24, dadurch **gekennzeichnet,** dass die Oxidation die 17α-Hydroxylierung von Progesteron ist.

27. Verfahren nach Anspruch 24, dadurch **gekennzeichnet,** dass mindestens zwei Oxidationen der Gruppe an dem gleichen Substratmolekül in einer Stufe durchgeführt werden.

## Revendications

1. Utilisation d'une cassette d'expression dans des systèmes multigéniques pour la réalisation de transformations multi-étapes comme le montre la figure 1, dans laquelle ladite cassette d'expression est opérationnelle chez un hôte recombinant non mammifère, caractérisée en ce que la cassette d'expression comprend une séquence d'ADN codante hétérologue codant pour une protéine, qui est fonctionnelle, seule ou en coopération avec une ou plusieurs protéines supplémentaires, pour catalyser une étape d'oxydation dans la voie biologique de transformation de cholestérol en hydrocortisone, laquelle étape est choisie dans le groupe constitué de :
la transformation de cholestérol en pregnénolone ;
la transformation de pregnénolone en progestérone ;
la transformation de progestérone en 17α-hydroxyprogestérone ;
la transformation de 17α-hydroxyprogestérone en cortexolone, et
la transformation de cortexolone en hydrocortisone,
et les séquences de contrôle correspondantes, efficaces chez ledit hôte.

2. Utilisation d'une cassette d'expression selon la revendication 1, caractérisée en ce que la protéine est choisie dans le groupe constitué de :
l'enzyme de clivage de la chaîne latérale (P₄₅₀SCC) ;
l'adrénodoxine (ADX) ;
l'adrénodoxine réductase (ADR) ;
la 3β-hydroxystéroïde déshydrogénase/isomérase (3β-HSD) ;
la stéroïde-17α-hydroxylase (P₄₅₀17α) ;
la NADPH cytochrome P₄₅₀ réductase (RED) ;
la stéroïde-21-hydroxylase (P₄₅₀C21), et
la stéroïde-11β-hydroxylase (P₄₅₀11β) .

3. Utilisation d'une cassette d'expression selon la revendication 2, caractérisée en ce que la séquence d'ADN codante hétérologue est issue d'espèces bovines.

4. Utilisation d'une cassette d'expression pGBSCC-5 selon la revendication 3, caractérisée en ce que l'ADN hétérologue code pour l'enzyme P₄₅₀SCC et en ce que la cassette d'expression présentée à la figure 8 est opérationnelle dans les espèces de Bacillus.

5. Utilisation d'une cassette d'expression pGBSCC-17 selon la revendication 3, caractérisée en ce que l'ADN hétérologue code pour l'enzyme P₄₅₀SCC et en ce que la cassette d'expression présentée à la figure 10 est opérationnelle chez E. Coli.

6. Utilisation d'une cassette d'expression selon la revendication 3, caractérisée en ce que l'ADN hétérologue code pour l'enzyme P₄₅₀SCC et en ce que la cassette d'expression opérationnelle chez Kluyveromyces lactis est choisie dans le groupe constitué de pGBSCC-7 présenté à la figure 14, pGBSCC-12 présenté à la figure 18 et pGBSCC-15 présenté à la figure 20 ou que la cassette d'expression opérationnelle chez Saccharomyces cerevisiae est choisie dans le groupe constitué de pGBSCC-10 présenté à la figure 17, pGBSCC-13 présenté à la figure 19 et pGBSCC-16 présenté à la figure 21.

7. Utilisation d'une cassette d'expression pGB17α-5 selon la revendication 3, caractérisée en ce que l'ADN hétérologue code pour l'enzyme P₄₅₀17α et que la cassette d'expression présentée à la figure 24 est opérationnelle chez Kluyveromyces lactis.

8. Utilisation d'une cassette d'expression pGB17α-8 selon la revendication 3, caractérisée en ce que l'ADN hétérologue code pour l'enzyme P₄₅₀17α et que la cassette d'expression présentée à la figure 27 est opérationnelle chez les espèces de Bacillus.

9. Utilisation d'une cassette d'expression pGB11β-4 selon la revendication 3, caractérisée en ce que l'ADN hétérologue code pour l'enzyme P₄₅₀11β et que la cassette d'expression présentée à la figure 37 est opérationnelle chez Kluyveromyces lactis.

10. Cassette d'expression, opérationnelle chez un hôte recombinant non mammifère, comprenant une séquence d'ADN codante hétérologue codant pour une protéine, qui est fonctionnelle, seule ou en coopération avec une ou plusieurs protéines supplémentaires, pour catalyser une étape d'oxydation dans la voie biologique de transformation de cholestérol en hydrocortisone, laquelle étape est choisie dans le groupe constitué de :
la transformation de cholestérol en pregnénolone ;
la transformation de pregnénolone en progestérone ;
la transformation de progestérone en 17α-hydroxyprogestérone ;
la transformation de 17α-hydroxyprogestérone en cortexolone, et
la transformation de cortexolone en hydrocortisone,
et les séquences de contrôle correspondantes, efficaces chez ledit hôte, caractérisée en ce que la protéine est choisie dans le groupe constitué de :
l'enzyme de clivage de la chaîne latérale (P₄₅₀SCC) ;
l'adrénodoxine (ADX) ;
l'adrénodoxine réductase (ADR) ;
la 3β-hydroxystéroïde déshydrogénase/isomérase (3β-HSD) ;
la stéroïde-17α-hydroxylase (P₄₅₀17α) ;
la NADPH cytochrome P₄₅₀ réductase (RED) ;
la stéroïde-21-hydroxylase (P₄₅₀C21), et
la stéroïde-11β-hydroxylase (P₄₅₀11β),
et en ce que l'ADN hétérologue code pour au moins une protéine supplémentaire dudit groupe de protéines.

11. Cassette d'expression selon la revendication 10, opérationnelle chez un hôte recombinant non mammifère, comprenant une séquence d'ADN codante hétérologue codant pour une protéine, qui est fonctionnelle , seule ou en coopération avec une ou plusieurs protéines supplémentaires, pour catalyser une étape d'oxydation dans la voie biologique de transformation de cholestérol en hydrocortisone, laquelle étape est choisie dans le groupe constitue de :
la transformation de cholestérol en pregnénolone ;
la transformation de pregnénolone en progestérone ;
la transformation de progestérone en 17α-hydroxyprogestérone ;
la transformation de 17α-hydroxyprogestérone en cortexolone, et
la transformation de cortexolone en hydrocortisone,
et les séquences de contrôle correspondantes, efficaces chez ledit hôte, caractérisée en ce que la protéine est choisie dans le groupe constitué de :
l'enzyme de clivage de la chaîne latérale (P₄₅₀SCC) ;
l'adrénodoxine (ADX) ;
l'adrénodoxine réductase (ADR) ;
la 3β-hydroxystéroïde déshydrogénase/isomérase (3β-HSD) ;
la stéroïde-17α-hydroxylase (P₄₅₀17α) ;
la NADPH cytochrome P₄₅₀ réductase (RED) ;
la stéroïde-21-hydroxylase (P₄₅₀C21), et
la stéroïde-11β-hydroxylase (P₄₅₀11β),
et en ce que l'ADN hétérologue codant pour ladite protéine supplémentaire est un ADN hétérologue supplémentaire avec ses propres séquences de contrôle efficaces codant pour une protéine dudit groupe de protéines.

12. Cassette d'expression selon la revendication 10 ou 11, caractérisée en ce que l'ADN hétérologue code pour la P₄₅₀SCC bovine et l'ADX bovine.

13. Cassette d'expression opérationnelle chez un hôte recombinant non mammifère, comprenant une séquence d'ADN codante hétérologue codant pour au moins deux protéines, qui sont fonctionnelles, seules ou en coopération avec une ou plusieurs protéines supplémentaires, pour catalyser au moins deux étapes d'oxydation choisies dans le groupe constitué de :
la transformation de cholestérol en pregnénolone ;
la transformation de pregnénolone en progestérone ;
la transformation de progestérone en 17α-hydroxyprogestérone ;
la transformation de 17α-hydroxyprogestérone en cortexolone, et
la transformation de cortexolone en hydrocortisone,
et les séquences de contrôle correspondantes, efficaces chez ledit hôte.

14. Cassette d'expression selon la revendication 13, opérationnelle chez un hôte recombinant non mammifère, comprenant une séquence d'ADN codante hétérologue codant pour au moins deux protéines, qui sont fonctionnelles, seules ou en coopération avec une ou plusieurs protéines supplémentaires, pour catalyser au moins deux étapes d'oxydation choisies dans le groupe constitué de :
la transformation de cholestérol en pregnénolone ;
la transformation de pregnénolone en progestérone ;
la transformation de progestérone en 17α-hydroxyprogestérone ;
la transformation de 17α-hydroxyprogestérone en cortexolone, et
la transformation de cortexolone en hydrocortisone,
caractérisée en ce que l'ADN hétérologue codant pour la deuxième protéine possède ses propres séquences de contrôle efficaces.

15. Cassette d'expression selon la revendication 13 ou 14, caractérisée en ce que la protéine est choisie dans le groupe constitué de :
l'enzyme de clivage de la chaîne latérale (P₄₅₀SCC) ;
l'adrénodoxine (ADX) ;
l'adrénodoxine réductase (ADR) ;
la 3β-hydroxystéroïde déshydrogénase/isomérase (3β-HSD) ;
la stéroïde-17α-hydroxylase (P₄₅₀17α) ;
la NADPH cytochrome P₄₅₀ réductase (RED) ;
la stéroïde-21-hydroxylase (P₄₅₀C21), et
la stéroïde-11β-hydroxylase (P₄₅₀11β) .

16. Cassette d'expression selon la revendication 15, caractérisée en ce que les séquences d'ADN codantes hétérologues sont issues d'espèces bovines.

17. Cellule hôte recombinante et la descendance de celle-ci comprenant des cellules de micro-organismes, de plantes ou d'animaux non mammifères et contenant une cassette d'expression à ADN hétérologue, caractérisée en ce que la cassette d'expression est définie selon l'une quelconque des revendications 10-16.

18. Cellule hôte recombinante et la descendance de celle-ci selon la revendication 17, caractérisée en ce que l'hôte est un micro-organisme.

19. Cellule hôte recombinante et la descendance de celle-ci selon la revendication 18, caractérisée en ce que l'hôte est une espèce de Saccharomyces, Kluyveromyces ou Bacillus ou est Escherichia coli.

20. Cellule hôte recombinante et la descendance de celle-ci selon l'une quelconque des revendications 17-19 et contenant au moins deux cassettes d'expression telles que définies dans l'une quelconque des revendications 10-16.

21. Procédé de préparation d'un mélange de protéines exogènes par une cellule recombinante dans un milieu nutritif dans des conditions permettant aux enzymes d'être formés et accumulés dans la culture,
caractérisé en ce que la cellule recombinante est une cellule hôte recombinante telle que définie dans la revendication 20.

22. Procédé d'oxydation biochimique sélective in vitro, lequel procédé comprend :
l'incubation du composé à oxyder en présence de protéines dans des conditions permettant ladite oxydation et l'accumulation du composé oxydé dans le liquide de culture, puis la récupération du composé oxydé, caractérisé en ce que les protéines sont produites par le procédé de la revendication 21.

23. Procédé d'oxydation d'un composé sélectionné, lequel procédé comprend :
la culture de cellules recombinantes en présence dudit composé dans des conditions dans lesquelles l'oxydation recherchée se produit et le composé oxydé s'accumule dans le liquide de culture, puis la récupération du composé oxydé, caractérisé en ce que les cellules recombinantes sont les cellules hôtes recombinantes de l'une quelconque des revendications 17-20.

24. Procédé selon la revendication 22 ou 23,
caractérisé en ce que l'oxydation est choisie parmi le groupe constitué par :
le clivage de la chaîne latérale d'un composé stérol en pregnénolone ;
la transformation de pregnénolone en progestérone ;
la transformation de progestérone en 17α-hydroxyprogestérone ;
la transformation de 17α-hydroxyprogestérone en cortexolone, et
la transformation de cortexolone en hydrocortisone.

25. Procédé selon la revendication 24, caractérisé en ce que l'oxydation est le clivage de la chaîne latérale d'un composé stérol conduisant à la pregnénolone.

26. Procédé selon la revendication 24, caractérisé en ce que l'oxydation est la 17α-hydroxylation de la progestérone.

27. Procédé selon la revendication 24, caractérisé en ce qu'au moins deux oxydations dudit groupe sont réalisées sur la même molécule substrat en une étape.
